# EUROPEAN PATENT APPLICATION

(11) **EP 4 796 036 A1**
(43) Date of publication of application: **26.08.2026**
(21) Application number: 24879436.4
(22) Date of filing: 28.08.2024
(51) Int. Cl.: A61B 5/30, A61B 5/256, A61B 5/31

(54) **BIOLOGICAL SIGNAL DETECTION DEVICE AND BIOLOGICAL SIGNAL DETECTION SYSTEM**

(30) Priority: 19.10.2023 JP 2023179964; 20.05.2024 JP 2024081761
(71) Applicant: Sony Group Corporation, Tokyo 108-0075 (JP)
(72) Inventor: YOSHIFUJI, Kazunari, Tokyo 108-0075 (JP); KATSUHARA, Mao, Tokyo 108-0075 (JP); SASAKI, Ryo, Tokyo 108-0075 (JP)
(74) Representative: D Young & Co LLP
(86) International application number: PCT/JP2024/030689
(87) International publication number: WO 2025/084008

(57) **Abstract**

A biological signal detection device according to an embodiment of the present disclosure includes: a first electrode and a second electrode that are configured to be in contact with a living body; a first signal line that is electrically coupled to the first electrode; a second signal line that is electrically coupled to the second electrode; a first resistor that is electrically coupled between the first signal line or the second signal line and a first potential line; and a first capacitor that is electrically coupled between the first signal line or the second signal line and the first potential line.

## Description

### Technical Field

The present disclosure relates to a biological signal detection device and a biological signal detection system.

### Background Art

A heart rate and electrocardiogram monitor has been proposed that includes a positive electrode, a reference electrode, and a negative electrode arranged to protrude on a surface that comes into contact with a living body (a human body, an animal body, etc.), and receives a biological signal (PTL 1).

### Citation List

### Patent Literature

PTL 1: Japanese Unexamined Patent Application Publication No. 2015-77270

### Summary of the Invention

In a device that detects a biological signal, it is desirable to reduce the number of electrodes.

It is desirable to provide a biological signal detection device that makes it possible to reduce the number of electrodes.

A biological signal detection device according to an embodiment of the present disclosure includes: a first electrode and a second electrode that are configured to be in contact with a living body; a first signal line that is electrically coupled to the first electrode; a second signal line that is electrically coupled to the second electrode; a first resistor that is electrically coupled between the first signal line or the second signal line and a first potential line; and a first capacitor that is electrically coupled between the first signal line or the second signal line and the first potential line.

A biological signal detection system according to an embodiment of the present disclosure includes: a measurement circuit including a first electrode and a second electrode that are configured to be in contact with a living body, a first signal line that is electrically coupled to the first electrode, a second signal line that is electrically coupled to the second electrode, a first resistor that is electrically coupled between the first signal line or the second signal line and a first potential line, and a first capacitor that is electrically coupled between the first signal line or the second signal line and the first potential line, the measurement circuit being configured to output a first signal based on a potential of the first signal line and a potential of the second signal line; and a signal processing circuit configured to generate a signal related to the living body, on the basis of the first signal outputted by the measurement circuit.

### Brief Description of Drawings

[FIG. 1] FIG. 1 is a diagram illustrating an example of a schematic configuration of a biological signal detection device according to a first embodiment of the present disclosure.
[FIG. 2] FIG. 2 is a diagram illustrating a configuration example of a measurer according to the first embodiment of the present disclosure.
[FIG. 3] FIG. 3 is a diagram illustrating another configuration example of the measurer according to the first embodiment of the present disclosure.
[FIG. 4] FIG. 4 is a diagram illustrating another configuration example of the measurer according to the first embodiment of the present disclosure.
[FIG. 5] FIG. 5 is a diagram for describing a configuration example of the measurer according to the first embodiment of the present disclosure.
[FIG. 6] FIG. 6 is a diagram illustrating a configuration example of the biological signal detection device according to the first embodiment of the present disclosure.
[FIG. 7] FIG. 7 is a diagram illustrating a configuration example of the biological signal detection device according to the first embodiment of the present disclosure.
[FIG. 8A] FIG. 8A is a diagram illustrating a configuration example of the biological signal detection device according to the first embodiment of the present disclosure.
[FIG. 8B] FIG. 8B is a diagram illustrating a configuration example of the biological signal detection device according to the first embodiment of the present disclosure.
[FIG. 9] FIG. 9 is a diagram for describing a configuration example of the biological signal detection device according to the first embodiment of the present disclosure.
[FIG. 10] FIG. 10 is a diagram for describing a configuration example of a measurer according to Modification example 1 of the present disclosure.
[FIG. 11A] FIG. 11A is a diagram for describing another configuration example of the measurer according to Modification example 1 of the present disclosure.
[FIG. 11B] FIG. 11B is a diagram for describing another configuration example of the measurer according to Modification example 1 of the present disclosure.
[FIG. 12] FIG. 12 is a diagram for describing a configuration example of a measurer according to Modification example 2 of the present disclosure.
[FIG. 13A] FIG. 13A is a diagram for describing another configuration example of the measurer according to Modification example 2 of the present disclosure.
[FIG. 13B] FIG. 13B is a diagram for describing another configuration example of the measurer according to Modification example 2 of the present disclosure.
[FIG. 14] FIG. 14 is a diagram for describing a configuration example of a measurer according to Modification example 3 of the present disclosure.
[FIG. 15A] FIG. 15A is a diagram for describing another configuration example of the measurer according to Modification example 3 of the present disclosure.
[FIG. 15B] FIG. 15B is a diagram for describing another configuration example of the measurer according to Modification example 3 of the present disclosure.
[FIG. 16A] FIG. 16A is a diagram for describing a configuration example of a measurer according to Modification example 4 of the present disclosure.
[FIG. 16B] FIG. 16B is a diagram for describing a configuration example of the measurer according to Modification example 4 of the present disclosure.
[FIG. 16C] FIG. 16C is a diagram for describing a configuration example of the measurer according to Modification example 4 of the present disclosure.
[FIG. 17A] FIG. 17A is a diagram for describing another configuration example of the measurer according to Modification example 4 of the present disclosure.
[FIG. 17B] FIG. 17B is a diagram for describing another configuration example of the measurer according to Modification example 4 of the present disclosure.
[FIG. 17C] FIG. 17C is a diagram for describing another configuration example of the measurer according to Modification example 4 of the present disclosure.
[FIG. 18] FIG. 18 is a diagram for describing a configuration example of a measurer according to Modification example 5 of the present disclosure.
[FIG. 19] FIG. 19 is a diagram for describing a configuration example of a measurer according to Modification example 6 of the present disclosure.
[FIG. 20] FIG. 20 is a diagram illustrating a configuration example of a measurer according to a second embodiment of the present disclosure.
[FIG. 21] FIG. 21 is a diagram illustrating a configuration example of the measurer according to the second embodiment of the present disclosure.
[FIG. 22] FIG. 22 is a diagram illustrating a configuration example of the measurer according to the second embodiment of the present disclosure.
[FIG. 23] FIG. 23 is a diagram for describing another configuration example of the measurer according to the second embodiment of the present disclosure.
[FIG. 24A] FIG. 24A is a diagram for describing a configuration example of a measurer according to Modification example 7 of the present disclosure.
[FIG. 24B] FIG. 24B is a diagram for describing a configuration example of the measurer according to Modification example 7 of the present disclosure.
[FIG. 25A] FIG. 25A is a diagram for describing another configuration example of the measurer according to Modification example 7 of the present disclosure.
[FIG. 25B] FIG. 25B is a diagram for describing another configuration example of the measurer according to Modification example 7 of the present disclosure.
[FIG. 26A] FIG. 26A is a diagram for describing another configuration example of the measurer according to Modification example 7 of the present disclosure.
[FIG. 26B] FIG. 26B is a diagram for describing another configuration example of the measurer according to Modification example 7 of the present disclosure.
[FIG. 27A] FIG. 27A is a diagram for describing a configuration example of a measurer according to Modification example 8 of the present disclosure.
[FIG. 27B] FIG. 27B is a diagram for describing the configuration example of the measurer according to Modification example 8 of the present disclosure.

### Modes for Carrying Out the Invention

Some embodiments of the present disclosure are described below in detail with reference to the drawings. It is to be noted that description is given in the following order.
0. Background
1. First Embodiment
2. Modification Examples
   2-1. Modification Example 1
   2-2. Modification Example 2
   2-3. Modification Example 3
   2-4. Modification Example 4
   2-5. Modification Example 5
   2-6. Modification Example 6
3. Second Embodiment
4. Modification Examples
   4-1. Modification Example 7
   4-2. Modification Example 8

### <0. Background>

Some of wearable devices with a biological measurement function perform biological measurement on users in their lives, record heart rates, and propose actions corresponding to measurement results. It is also possible to use a biological signal in combination with context information to estimate a user's internal state and also to support the user's action. In a case of a wearable device with a bioelectric potential measurement function, it is desirable that, in wearing the wearable device, an electrode (a bioelectrode) come into contact with a measurement site at the same time as the wearing of the wearable device, without additional work or complicated operations.

### <1. First Embodiment>

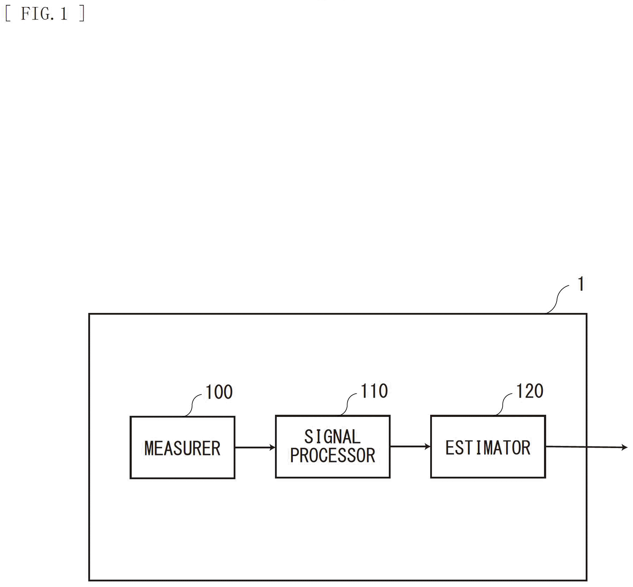

FIG. 1 is a diagram illustrating an example of a schematic configuration of a biological signal detection device according to a first embodiment of the present disclosure. The biological signal detection device 1 is a device configured to perform bioelectric potential measurement, and may be referred to as a bioelectric potential measurement device. The biological signal detection device 1 is implemented to execute biological measurement of a brainwave (EEG: Electroencephalography), a cardiac signal (ECG: Electrocardiogram), or the like.

Examples of a biological signal include a brainwave that is a signal associated with cerebral activity, a cardiac signal associated with cardiac activity, and a myoelectric signal associated with muscular activity. In the biological signal detection device 1 according to the present embodiment, a bioelectric potential signal such as a brainwave, a cardiac signal, or a myoelectric potential is measured as an electric signal. A bioelectric potential at a position of an electrode mounted on a body surface of a user is measured.

The biological signal detection device 1 is applicable to, for example, an electronic apparatus to be worn by the user. The biological signal detection device 1 may be applied to the electronic apparatus that is wearable on a body including an ear, a head, a face, a neck, a hand, a wrist, an arm, a foot, a chest, etc. The biological signal detection device 1 may be implemented as a device to be mounted on wearable devices including, for example, earphones, headphones, and eyewear.

The biological signal detection device 1 includes a measurer 100 and a signal processor 110. The biological signal detection device 1 may also include an estimator 120, as in the example illustrated in FIG. 1. The estimator 120 may be provided in the signal processor 110 or may be provided separately from the signal processor 110. The biological signal detection device 1 (or the measurer 100) may be configured, for example, as a sensor (a biological sensor) that is configured to acquire the biological signal.

The measurer 100 is a measurement circuit and is configured to generate the biological signal. The measurer 100 includes a plurality of electrodes for detecting a potential, which will be described later. The measurer 100 (the measurement circuit) is, for example, configured to execute brainwave measurement and configured to detect a brainwave signal as the biological signal. The measurer 100 may acquire the biological signal of the user and output the biological signal to the signal processor 110.

The signal processor 110 is a signal processing circuit and is configured to execute signal processing (information processing). The signal processor 110 includes, for example, a processor and memories (ROM, RAM and the like), and is configured to perform various kinds of signal processing. The signal processor 110 may read and execute a program incorporated therein, and perform the signal processing (the information processing).

The signal processor 110 is configured to execute the signal processing on a signal to be inputted. The signal processor 110 (the signal processing circuit) includes, for example, a circuit that performs various kinds of signal processing on the biological signal. The signal processor 110 may perform various kinds of signal processing including, for example, a process of reducing noises, a frequency-analysis process, and a normalization process on the biological signal to be outputted from the measurer 100.

The estimator 120 is configured to execute a process of estimating a state of the living body with use of the biological signal. The estimator 120 includes, for example, a processor, a memory, and the like. The estimator 120 may perform, for example, a process of calculating a feature quantity using the biological signal after being subjected to a noise-reduction process.

The estimator 120 extracts the feature quantity by performing, for example, a Fast Fourier Transform (FFT) process or a wavelet analysis process on the biological signal. Examples of the feature quantity include a θ-wave component (4 Hz to 7 Hz), a α-wave component (8 Hz to 12 Hz), and a β-wave component (13 Hz to 30 Hz) included in the brainwave signal.

The estimator 120 performs a process of estimating the state of the user on the basis of a result of the calculation of the feature quantity. The estimator 120 estimates the state such as whether or not the user is relaxed or whether or not the living body is concentrating, for example, on the basis of the α-wave component and the β-wave component. The estimator 120 may be referred to as a determiner configured to determine the state of the user.

As another example, the estimator 120 may estimate a sleeping state of the user with use of the biological signal related to the brainwave. Further, for example, the estimator 120 may estimate a heart rate with use of the biological signal related to the cardiac signal. The estimator 120 may estimate an emotion of the user by analyzing the biological signal.

The biological signal detection device 1 may be configured to acquire information (context information) related to a situation of the user, and execute a process of estimating the state of the living body, a process of proposing (presenting) an action to the user, and the like. The context information includes, for example, information related to an action (a motion) of the user, information related to a position of the user, information related to a time, and information related to a sound.

The biological signal detection device 1 (or the measurer 100) may include, for example, as a sensor unit, various sensors configured to acquire the context information, such as an acceleration sensor, a gyro sensor (an angular velocity sensor), a GPS sensor, and a sound sensor. The sensor unit may be provided in the biological signal detection device 1 or may be provided separately from the biological signal detection device 1.

The estimator 120 is configured to generate information indicating the state of the living body (hereinafter referred to as state information). The estimator 120 may generate and output the state information as an estimation result. The state information includes, for example, information indicating whether or not the living body is in a relaxed state, information indicating whether or not the living body is in a sleeping state, information indicating a psychological state such as an emotion of the living body, and information indicating a heart rate.

The state information may be used, for example, for displaying an image indicating the state of the living body, outputting a sound indicating the state of the living body, and the like. The biological signal detection device 1 may include, for example, a display unit configured to display an image based on the state information. The display unit is, for example, an organic EL display, a liquid crystal display, or the like. The display unit may include a touch panel.

The biological signal detection device 1 may include a sound output unit, such as a speaker, that is configured to output a sound (a music piece (BGM), a sound effect, or the like) based on the state information. The biological signal detection device 1 may be configured to apply a vibration generated by, for example, a vibration function to the user on the basis of the state information.

The biological signal detection device 1 includes, for example, a presentation unit (the display unit, the sound output unit, or the like) configured to present (provide) an image, a sound, a vibration, or the like to the user. The biological signal detection device 1 may be configured to decide (determine) an action recommended to the user with use of the biological information and the context information. The biological signal detection device 1 may cause the display unit to display an image that prompts the recommended action, and may cause the sound output unit to output a voice message that prompts the recommended action.

It is to be noted that the signal processor 110, the estimator 120, or both may be provided in an external device that is provided outside the biological signal detection device 1. Examples of the external device include an electronic apparatus that is a terminal device (a terminal) to be used by the user, a server (for example, a cloud server), and the like. Examples of the electronic apparatus include a smartphone, a tablet terminal, a wearable terminal, a computer, and another information processing device.

A combination of the biological signal detection device 1 and the external device may also be referred to as a biological signal detection device. The biological signal detection device 1 and the external device may transmit and receive a signal (information) via wireless or wired communication. For example, the biological signal detection device 1 is configured to be coupled to a network and to communicate with the external device. A combination of the biological signal detection device 1 and the external device coupled to each other via the network may also be referred to as a biological signal detection device or a biological signal detection system.

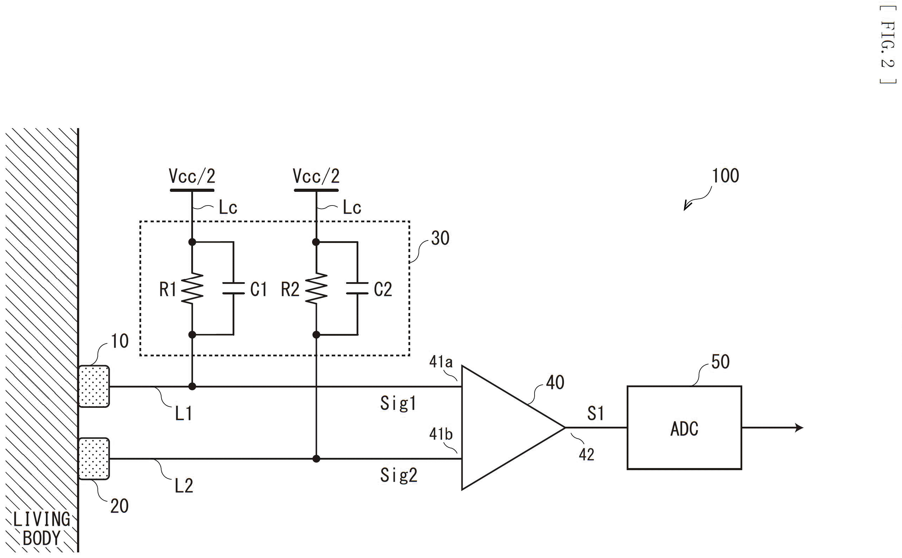

FIG. 2 is a diagram illustrating a configuration example of the measurer according to the first embodiment of the present disclosure. The measurer 100 includes an electrode to be used for detecting a potential, and is configured to measure the bioelectric potential. As in the example illustrated in FIG. 2, the measurer 100 includes, for example, two electrodes (referred to as a signal electrode 10 and a reference electrode 20), a connection circuit 30, an amplifier circuit 40, and an AD converter circuit 50.

The signal electrode 10 and the reference electrode 20 are each configured to be in contact with the living body. The signal electrode 10 and the reference electrode 20 are provided away from each other, and come into contact with respective positions that are different from each other. The signal electrode 10 is configured to be disposed at any position from which the biological signal is to be acquired. The reference electrode 20 is configured to be disposed at any position including the vicinity of the signal electrode 10. The measurer 100 detects a potential (a voltage) of a surface of the living body with use of the signal electrode 10 and the reference electrode 20.

The signal electrode 10 and the reference electrode 20 each include an electrically conductive material. The signal electrode 10 and the reference electrode 20 each include, for example, silver/silver chloride (Ag/AgCl), aluminum (Al), copper (Cu), gold (Au), carbon (C), or the like. The signal electrode 10 and the reference electrode 20 may each include, for example, an electrically conductive and elastic resin material.

The signal electrode 10 and the reference electrode 20 may each include PEDOT:PSS. The signal electrode 10 and the reference electrode 20 may each include another material having a high affinity for the living body. Using a biocompatible material makes it possible to configure the signal electrode 10 and the reference electrode 20 as dry electrodes that use no paste or gel. It is to be noted that a shape of each of the signal electrode 10 and the reference electrode 20 is not particularly limited, and may be a circular shape, an oval shape, a polygonal shape, or another shape.

The connection circuit 30 is electrically coupled between the signal electrode 10 (or the reference electrode 20) and a reference potential line Lc. The reference potential line Lc is a wiring to which a predetermined potential (voltage) is applied. The connection circuit 30 includes, for example, an impedance element (a resistive impedance element, a capacitive impedance element, or the like) and has a predetermined impedance. The impedance element may include a passive element, or may include an active element.

The connection circuit 30 includes, for example, a resistor R1, a resistor R2, a capacitor C1, and a capacitor C2, as in the example illustrated in FIG. 2. Each of the resistor R1 and the resistor R2 is a resistive impedance element, and is a resistive body (a resistive member). The resistors R1 and R2 may be passive elements or may include active elements.

The resistor R1 and the resistor R2 may each include a transistor (e.g., a MOS transistor). The resistors R1 and R2 may each include, for example a diode-coupled transistor. The resistors R1 and R2 may each include a variable resistor.

Each of the capacitor C1 and the capacitor C2 is a capacitive impedance element, and is a capacitor (a condenser). The capacitor C1 and the capacitor C2 may be passive elements or may include active elements. The capacitors C1 and C2 may each include a capacitor such as a MOS capacitance or a MIM (Metal-Insulator-Metal) capacitance. The capacitors C1 and C2 may each include a variable capacitor.

The reference potential line Lc is, for example, a wiring to which a potential (an intermediate potential) between a potential of a power supply line and a ground potential is applied. For example, the reference potential line Lc is supplied with a voltage (a voltage value) that is half a power supply voltage Vcc, that is, Vcc/2. The reference potential line Lc serves as a potential line to which a potential corresponding to the potential (a voltage) of the power supply line of the measurer 100 is applied. It is to be noted that the reference potential line Lc may be a ground line (grounding line). For example, in a case where the reference potential line Lc is a ground line (grounding line), positive and negative power sources (e.g., +5 V/-5 V) are used as a power source of the circuit.

In the example illustrated in FIG. 2, the reference potential line Lc is a wiring to which the voltage Vcc/2 is applied. The reference potential line Lc may be a wiring to which the ground potential (grounding potential) is applied. For example, 0 V may be applied as a ground voltage (GND voltage) to the reference potential line Lc. The reference potential line Lc may be supplied with another specific voltage.

The resistor R1 and the capacitor C1 of the connection circuit 30 are, for example, electrically coupled between the signal electrode 10 and the reference potential line Lc. In the example illustrated in FIG. 2, the resistor R1 is electrically coupled between a signal line L1 electrically coupled to the signal electrode 10, and the reference potential line Lc. Further, the capacitor C1 is electrically coupled between the signal line L1 and the reference potential line Lc. The resistor R1 and the capacitor C1 are electrically coupled in parallel to each other between the signal line L1 and the reference potential line Lc.

The resistor R2 and the capacitor C2 are, for example, electrically coupled between the reference electrode 20 and the reference potential line Lc. In the example illustrated in FIG. 2, the resistor R2 is electrically coupled between a signal line L2 electrically coupled to the reference electrode 20, and the reference potential line Lc. Further, the capacitor C2 is electrically coupled between the signal line L2 and the reference potential line Lc. The resistor R2 and the capacitor C2 are electrically coupled in parallel to each other between the signal line L2 and the reference potential line Lc.

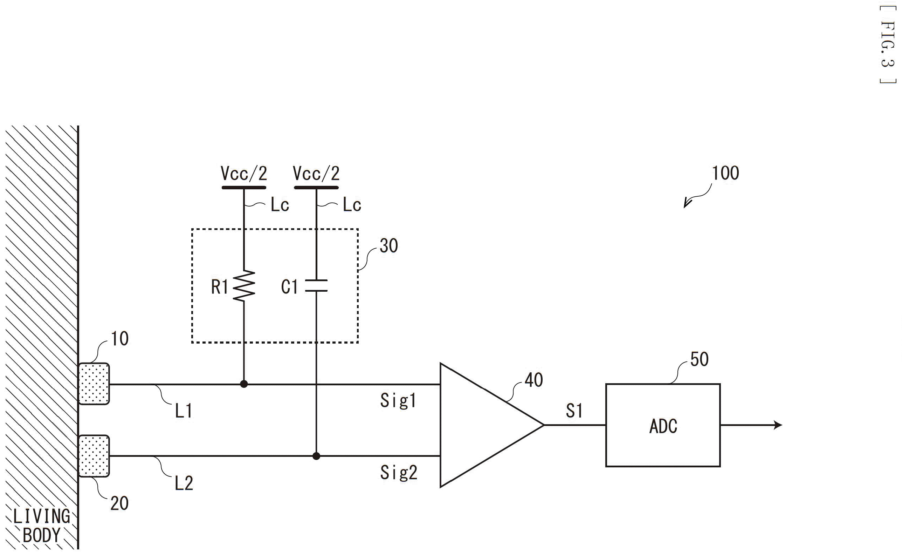

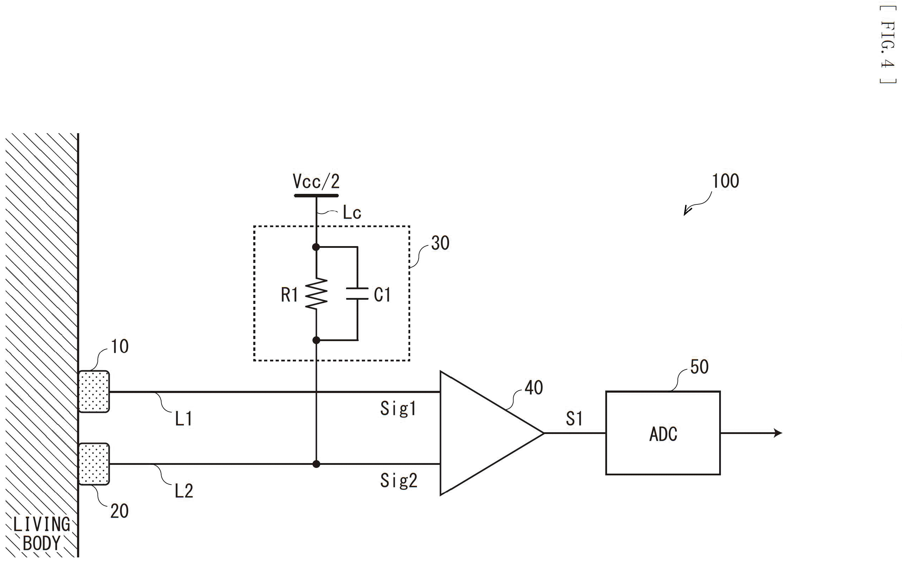

It is to be noted that a configuration of the connection circuit 30 is not limited to the example illustrated in FIG. 2, and may be changed as appropriate. The connection circuit 30 may have a configuration illustrated in FIG. 3 or FIG. 4. In the example illustrated in FIG. 3, the connection circuit 30 includes the resistor R1 electrically coupled between the signal line L1 and the reference potential line Lc, and the capacitor C1 electrically coupled between the signal line L2 and the reference potential line Lc. It is to be noted that the capacitor C1 may be electrically coupled between the signal line L1 and the reference potential line Lc, and the resistor R1 may be electrically coupled between the signal line L2 and the reference potential line Lc.

In the example illustrated in FIG. 4, the connection circuit 30 includes the resistor R1 and the capacitor C1 each electrically coupled between the signal line L2 and the reference potential line Lc. The resistor R1 and the capacitor C1 are coupled in parallel to each other. It is to be noted that the resistor R1 and the capacitor C1 may each be electrically coupled in parallel to each other between the signal line L1 and the reference potential line Lc.

The signal electrode 10 (or the reference electrode 20) and the reference potential line Lc are electrically coupled to each other via the connection circuit 30. The signal line L1, the signal line L2, or both are electrically coupled to the reference potential line Lc via the resistor or the capacitor of the connection circuit 30. This makes it possible to set a relative potential between the measurer 100 and the living body. For example, a relationship of a direct-current potential between a circuit of the biological signal detection device 1 and the living body is defined.

The signal electrode 10 comes into contact with a measurement site (a point to be measured) in actual use, and a potential of the contact site is applied, as in the examples schematically illustrated in FIGs. 2 to 4. The signal electrode 10 is disposed, for example, directly on an activity region, of the living body, in which the biological signal is to be measured. The signal electrode 10 is electrically coupled to the amplifier circuit 40, for example, and supplies a measurement signal Sig1 to the amplifier circuit 40. The measurement signal Sig1 is a signal corresponding to the potential (the bioelectric potential) of the contact site of the living body. The measurement signal Sig1 is a biological signal obtained by the signal electrode 10.

The reference electrode 20 comes into contact with the living body at a position that differs from the position of the signal electrode 10 in actual use, and a potential of the contact site is applied, as in the examples illustrated in FIGs. 2, 3, etc. The reference electrode 20 may be disposed at any position around the signal electrode 10, for example. The reference electrode 20 is electrically coupled to the amplifier circuit 40, for example, and supplies a measurement signal Sig2 to the amplifier circuit 40. The measurement signal Sig2 corresponds to the potential of the contact site of the living body. The measurement signal Sig2 is a biological signal obtained by the reference electrode 20.

The amplifier circuit 40 is configured to amplify a signal to be inputted. The amplifier circuit 40 includes, for example, a differential amplifier circuit (a differential amplifier) configured to amplify a signal. The amplifier circuit 40 (a differential amplifying unit) generates a biological signal S1 on the basis of, for example, a measurement signal obtained by the signal electrode 10 and a measurement signal obtained by the reference electrode 20. The amplifier circuit 40 may also be referred to as a signal detector (or a signal generator) configured to detect a biological signal.

In the examples illustrated in FIG. 2, etc., the measurement signal Sig1 is inputted to the amplifier circuit 40 via the signal line L1 by the signal electrode 10. Further, the measurement signal Sig2 is inputted to the amplifier circuit 40 via the signal line L2 by the reference electrode 20. The amplifier circuit 40 generates, for example, the biological signal S1 based on a difference between the measurement signal Sig1 and the measurement signal Sig2. The amplifier circuit 40 may generate the biological signal S1 corresponding to a difference between a potential of the measurement signal Sig1 and a potential of the measurement signal Sig2, and output the generated biological signal S1 to the AD converter circuit 50.

The amplifier circuit 40 includes, for example, an input section 41a, an input section 41b, and an output section 42, as illustrated in FIG. 2, and includes an amplifier circuit configured to amplify a signal. The input section 41a of the amplifier circuit 40 is a first input terminal, and is electrically coupled to the signal line L1. The measurement signal Sig1 is inputted to the input section 41a from the signal electrode 10 via the signal line L1. The input section 41b of the amplifier circuit 40 is a second input terminal, and is electrically coupled to the signal line L2. The measurement signal Sig2 is inputted to the input section 41b from the reference electrode 20 via the signal line L2. The amplifier circuit 40 may generate the biological signal S1, and output the biological signal S1 from the output section 42 to the AD converter circuit 50.

The AD converter circuit 50 is configured to convert an analog signal to be inputted into a digital signal. The AD converter circuit 50 is an ADC (Analog to Digital Converter). The biological signal S1 is inputted to the AD converter circuit 50 from the amplifier circuit 40, for example. The AD converter circuit 50 performs an AD conversion process on the biological signal S1 as the analog signal inputted from the amplifier circuit 40.

The AD converter circuit 50 (an AD converter) converts the biological signal S1 outputted by the amplifier circuit 40 into the digital signal. The AD converter circuit 50 may output the biological signal S1 converted into the digital signal to the signal processor 110 (see FIG. 1). The signal processor 110 acquires the biological signal S1 from the measurer 100, and performs a noise reduction process, a frequency analysis process, and the like on the biological signal S1. The estimator 120 may perform, for example, a process of generating the state information with use of the biological signal S1.

In the biological signal detection device 1 according to the present embodiment, the signal line L1 coupled to the signal electrode 10 (or the signal line L2 coupled to the reference electrode 20) and the reference potential line Lc are electrically coupled to each other via the connection circuit 30, as described above. Thus, the potential relationship between the circuit of the measurer 100 and the living body is set. This allows the measurer 100 to have a configuration that does not include a dedicated bioelectrode (referred to as a bias electrode, a ground electrode, a grounding electrode, or the like) for defining the potential relationship.

It is possible to reduce the number of electrodes of the measurer 100, as compared with a case of providing a ground electrode (a bias electrode) separately from the signal electrode 10 and the reference electrode 20. It is unnecessary to provide a dedicated ground electrode to be mounted on the living body, which makes it possible to reduce the number of electrodes to be brought into contact with the living body.

Accordingly, in the present embodiment, for example, it is possible to make it less difficult to bring the electrode into contact with the living body at the same time as the wearing of the wearable device. It is possible to allow for (ensure) contact of the signal electrode 10 and the reference electrode 20 with the living body in wearing the wearable device, thereby improving usability.

In the present embodiment, it is possible to accurately detect the biological signal by the signal electrode 10 and the reference electrode 20, even in a case of a very low signal level (e.g., an amplitude of 10 µV or less), such as the brainwave, a case where a waveform of the biological signal has to be acquired, etc.

Further, because shapes of ears differ among individuals, it is very difficult to allow for contact between the living body and the electrodes if there are three electrodes that necessitate contact for the bioelectric potential measurement. In the present embodiment, it is possible to reduce the electrodes that necessitate contact to two electrodes, making it possible to easily mount the electrodes.

A resistance value or a capacitance value of the impedance element of the connection circuit 30 may be set as appropriate depending on a frequency of the signal to be measured. For example, the resistance value of the resistive impedance element and the capacitance value of the capacitive impedance element are set to provide a cut-off frequency that is set in consideration of a necessary transmission band of an input signal.

The resistance value of the resistor and the capacitance value of the capacitor of the connection circuit 30 are set on the basis of, for example, a frequency of the signal (the biological signal) that is inputted to the signal line L1 (or the signal line L2). The connection circuit 30 has a cut-off frequency based on the resistance value of the resistor and the capacitance value of the capacitor, and may be referred to as a filtering circuit. The connection circuit 30 is, for example, a HPF (High Pass Filter) including the resistor and the capacitor.

The connection circuit 30 (the filtering circuit) may be configured to selectively transmit signals in a specific frequency band. A cut-off frequency fc of the connection circuit 30 is set on the basis of, for example, the frequency of the signal that is inputted to the signal line L1 (or the signal line L2). For example, resistance values of the resistors R1 and R2 and capacitance values of the capacitors C1 and C2 are adjusted to attenuate unnecessary frequency components and allow the measurement signals Sig1 and Sig2 in a predetermined frequency range to be transmitted to the amplifier circuit 40.

The resistance value of each of the resistors R1 and R2 and the capacitance value of each of the capacitors C1 and C2 are set on the basis of, for example, the frequency of the brainwave. The resistance values of the resistors R1 and R2, the capacitance values of the capacitors C1 and C2, the cut-off frequency fc, and the like are defined to allow for selective transmission of a signal in the frequency range of the brainwave, among signals that are inputted to the signal line L1 (or the signal line L2).

In the examples illustrated in FIGs. 2, 3, etc., the cut-off frequency fc of the connection circuit 30 (the filtering circuit) may be expressed by, for example, the following expression (1).
[Math. 1] ${f}_{c} = \frac{1}{2 {πR}_{1} {C}_{1}}$

An impedance Z1 of the connection circuit 30 may be expressed by the following expression (2) and the following expression (3). $\begin{matrix}{Z}_{1} = \frac{{R}_{1} ⋅ \frac{1}{{jωC}_{1}}}{{R}_{1} + \frac{1}{{jωC}_{1}}} = \frac{{R}_{1}}{{ω}^{2} {R}_{1}^{2} {C}_{1}^{2} + 1} + j \frac{{ωR}_{1}^{2} {C}_{1}}{{ω}^{2} {R}_{1}^{2} {C}_{1}^{2} + 1} & \left(2\right) \\ \left|{Z}_{1}\right| = \frac{1}{{ω}^{2} {R}_{1}^{2} {C}_{1}^{2} + 1} \sqrt{{R}_{1}^{2} + {\left({ωR}_{1}^{2}{C}_{1}\right)}^{2}} & \left(3\right)\end{matrix}$

Assuming an electroencephalograph, a case is considered where the frequency band to be transmitted is set to, for example, 0.5 Hz to 40 Hz. In this case, the connection circuit 30 may be configured to have, for example, the cut-off frequency fc ≤ 0.2 Hz. Further, for example, the connection circuit 30 may be configured to have the cut-off frequency fc ≤ 0.1 Hz, or may be configured to have the cut-off frequency fc ≤ 0.05 Hz.

The connection circuit 30 may be configured as a HPF including the resistor R1 and the capacitor C1. For example, in the example illustrated in FIG. 3, the resistor R1 (resistance value) =10 MΩ and the capacitor C1 (capacitance value) =1 µF may be set. In this case, the cut-off frequency fc = 0.016 Hz is obtained from the above-described expression (1).

In the example illustrated in FIG. 2, circuit constants may be set to make the resistor R1 and the resistor R2 have the same resistance value. Further, the capacitor C1 and the capacitor C2 may have the same capacitance value. For example, the connection circuit 30 may have circuit constants of R1 = R2 = 10 MΩ and C1 = C2 = 1 µF. In the example illustrated in FIG. 2, the connection circuit 30 includes the resistors R1 and R2 coupled in parallel and the capacitors C1 and C2 coupled in parallel. The connection circuit 30 may be regarded as a HPF including the resistor with a resistance value of 5 MΩ and the capacitor with a capacitance value of 2 µF.

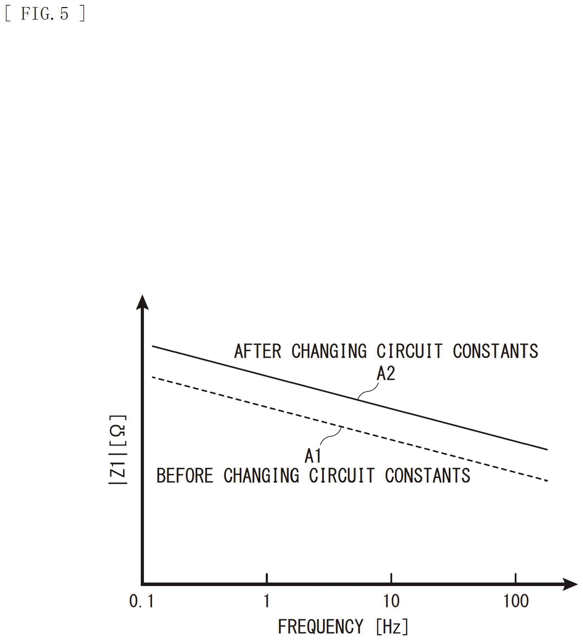

Depending on the circuit constants of the connection circuit 30 (e.g., in a case where R1 = R2 = 10 MΩ and C1 = C2 = 1 µF), the impedance Z1 can be low as indicated by a broken line A1 in FIG. 5, and there is a possibility that a desired amplitude (signal level) of the measurement signal Sig1 (or the measurement signal Sig2) is not obtained. In FIG. 5, the vertical axis represents a magnitude |Z1| of the impedance, and the horizontal axis represents the frequency.

Hence, in the connection circuit 30 illustrated in FIG. 2, R1 = R2 = 10 MΩ and C1 = C2 = 0.1 µF may be set. In this case, the connection circuit 30 may be regarded as a HPF including the resistor with a resistance value of 5 MΩ and the capacitor with a capacitance value of 0.2 µF. The cut-off frequency fc = 0.016 Hz is obtained from the above-described expression (1). Setting the circuit constants in this manner makes it possible to set a desired cut-off frequency, while ensuring the impedance Z1 as indicated by a solid line A2 in FIG. 5.

The technology according to the present disclosure is applicable to a variety of products. For example, the biological signal detection device and the measurer according to the present disclosure may be used for various electronic apparatuses with the biological measurement function. The biological signal detection device 1 and the measurer 100 according to the present disclosure may be applied to, for example, a wearable device such as an earphone device or a headphone device.

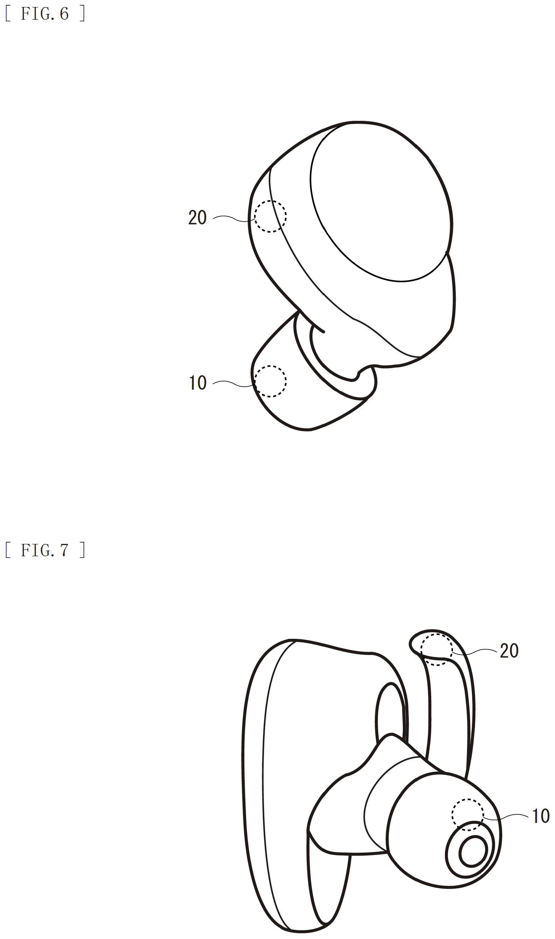

For example, the biological signal detection device 1 may be mounted on an earphone device such as a TWS (True Wireless Stereo), as in an example illustrated in FIG. 6 or FIG. 7. One of the signal electrode 10 and the reference electrode 20, for example, the signal electrode 10, may be shaped as an earpiece-type dry electrode, and disposed to come into contact with an ear hole in actual use. The other of the signal electrode 10 and the reference electrode 20, for example, the reference electrode 20, may be disposed to come into contact with another part of the ear in actual use.

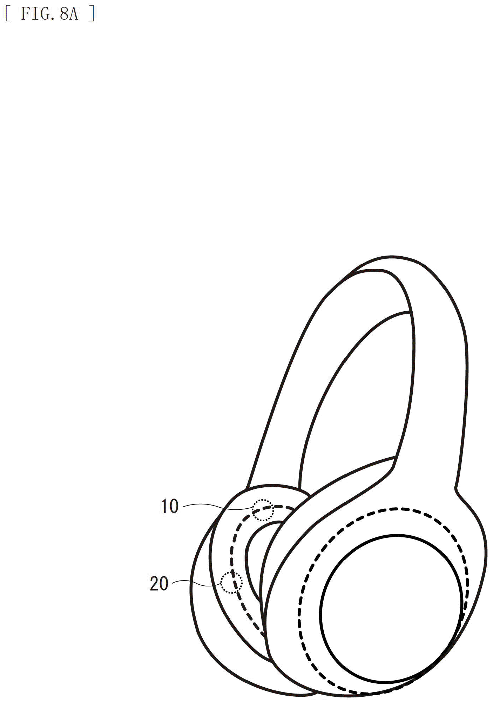

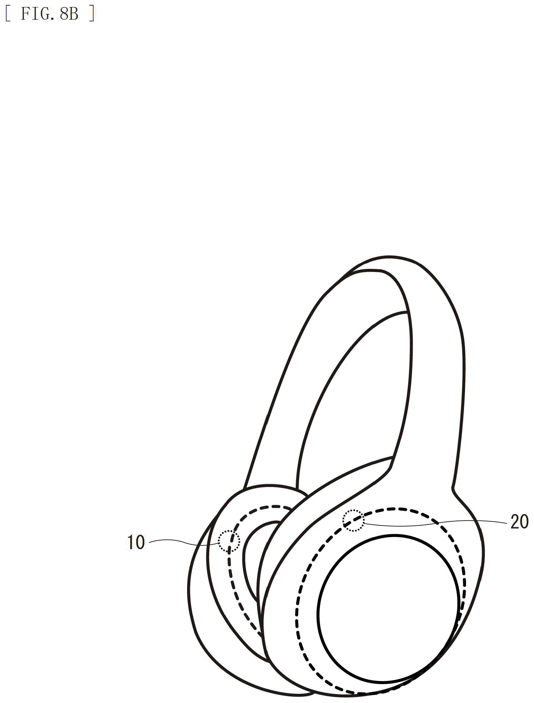

As another example, the biological signal detection device 1 may be applied to a headphone device, as in an example illustrated in FIG. 8A. For example, in a case of an overhead headphone, the signal electrode 10 and the reference electrode 20 may be mounted on a surface of an ear pad. Further, the signal electrode 10 may be provided on one ear pad, among two ear pads, and the reference electrode 20 may be provided on the other ear pad, as in an example schematically illustrated in FIG. 8B.

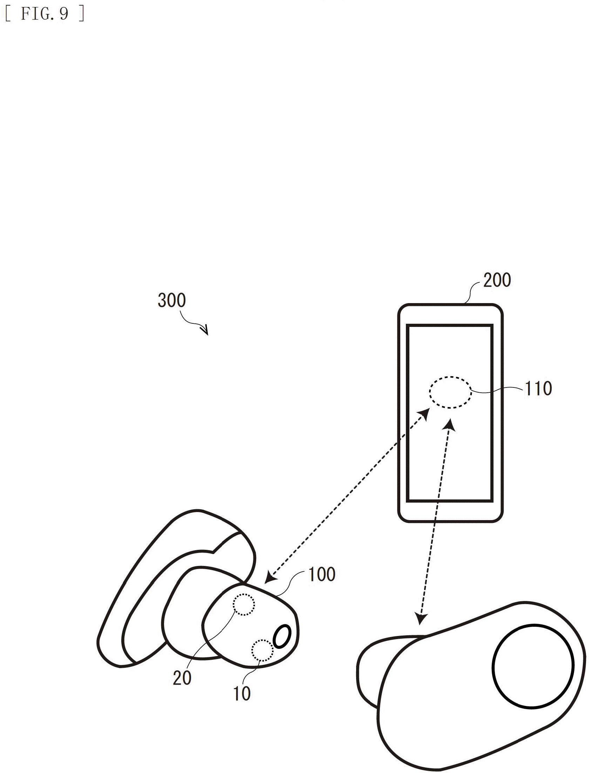

The signal processor 110 and the estimator 120 (also see FIG. 1) may be implemented in the wearable device (the earphone device, the headphone device, etc.). Further, the signal processor 110 and the estimator 120 may be provided in an electronic apparatus 200 that is provided outside the biological signal detection device 1, as in an example illustrated in FIG. 9. The electronic apparatus 200 is, for example, a terminal device to be used by the user. The electronic apparatus 200 may be a smartphone, a tablet terminal, a wearable terminal, a computer, or another information processing device.

As in the example illustrated in FIG. 9, the biological signal detection device 1 may be configured as a biological signal detection system 300 including: the measurer 100; the electronic apparatus 200 including the signal processor 110; and the like. The biological signal detection system 300 may include, for example, the estimator 120, the display unit, the sound output unit, and the like that have been described above. It is to be noted that the signal processor 110 and the estimator 120 may be integrated with each other. The signal processor 110 may include the estimator 120.

### <Workings and Effects>

The biological signal detection device (the biological signal detection device 1) according to the present embodiment includes: a first electrode and a second electrode (the signal electrode 10 and the reference electrode 20) that are configured to be in contact with the living body; a first signal line (e.g., the signal line L1) that is electrically coupled to the first electrode; a second signal line (the signal line L2) that is electrically coupled to the second electrode; a first resistor (e.g., the resistor R1) that is electrically coupled between the first signal line or the second signal line and a first potential line (the reference potential line Lc); and a first capacitor (the capacitor C1) that is electrically coupled between the first signal line or the second signal line and the first potential line.

In the biological signal detection device 1 according to the present embodiment, the resistor R1 that is electrically coupled between the signal line L1 (or the signal line L2) and the reference potential line Lc, and the capacitor C1 that is electrically coupled between the signal line L1 (or the signal line L2) and the reference potential line Lc are provided. This makes it possible to set the potential relationship between the biological signal detection device 1 and the living body. It is possible to achieve a biological signal detection device that makes it possible to reduce the number of electrodes.

Next, description is given of modification examples of the present disclosure. Hereinafter, components similar to those in the foregoing embodiment are denoted by the same reference numerals, and descriptions thereof are omitted as appropriate.

### <2. Modification Examples>

### <2-1. Modification Example 1>

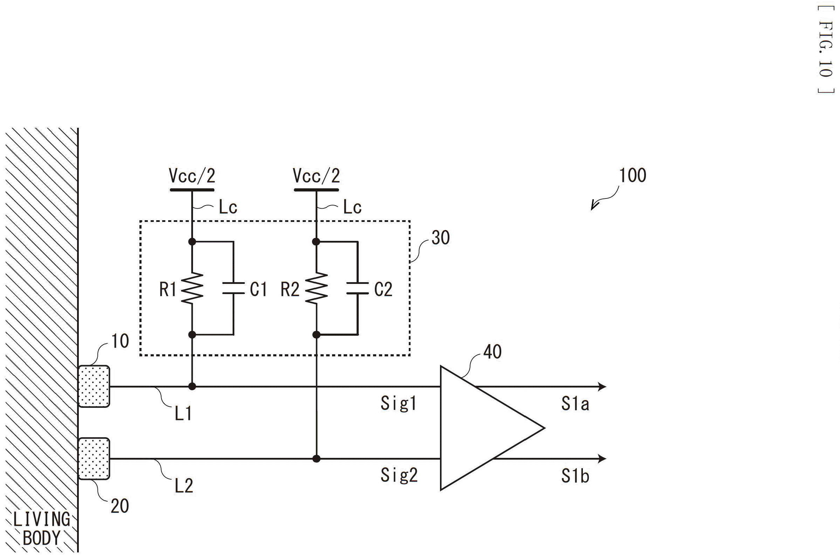

Although configuration examples of the measurer 100 have been described in the above-described embodiment, the configuration is merely an example, and the configuration of the measurer 100 is not limited to the above-described example. For example, the amplifier circuit 40 of the measurer 100 may be a single-output amplifier circuit or may be a differential-output amplifier circuit. FIG. 10 is a diagram for describing a configuration example of a measurer according to Modification example 1 of the present disclosure. The amplifier circuit 40 of the measurer 100 may be configured to output differential signals, as in the example illustrated in FIG. 10. The amplifier circuit 40 may be configured as a differential-output amplifier circuit.

The amplifier circuit 40 is configured to, for example, output a biological signal S1a and a biological signal S1b with use of the measurement signal Sig1 and the measurement signal Sig2 as differential input signals. The biological signal S1b is an inverted signal of the biological signal S1a. The amplifier circuit 40 is configured to generate the biological signal S1a and the biological signal S1b as the inverted signal of the biological signal S1a. The amplifier circuit 40 may, for example, generate the biological signals S1a and S1b based on the difference between the measurement signal Sig1 and the measurement signal Sig2, and output the biological signals S1a and S1b to the AD converter circuit 50.

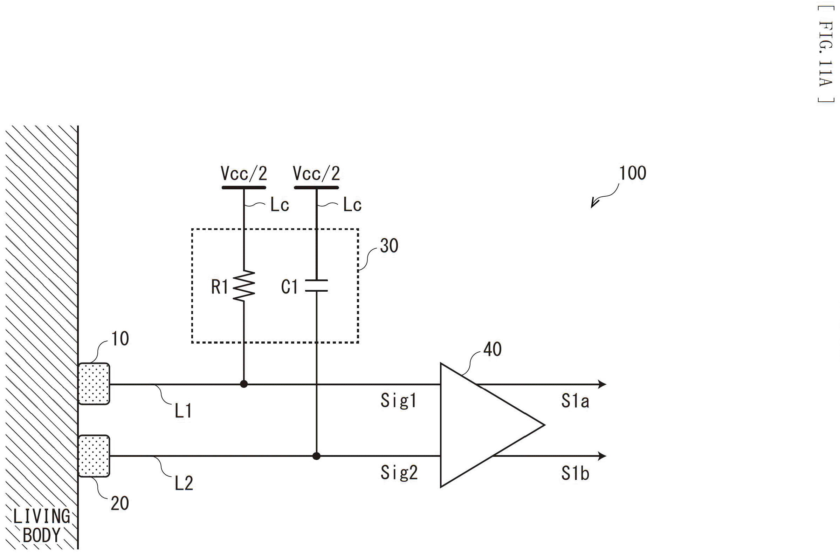

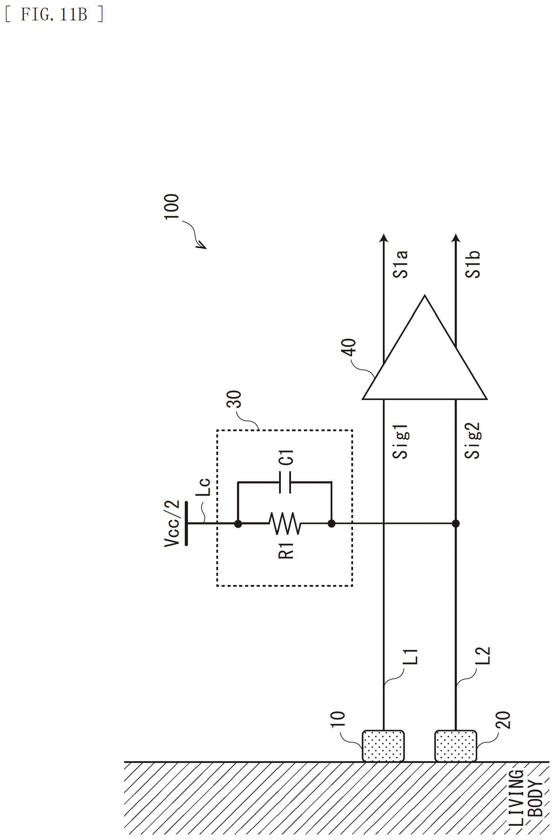

FIGs. 11A and 11B are each a diagram for describing another configuration example of the measurer according to Modification example 1. The connection circuit 30 of the measurer 100 may include the resistor R1 electrically coupled between the signal line L1 and the reference potential line Lc, and the capacitor C1 electrically coupled between the signal line L2 and the reference potential line Lc, as illustrated in FIG. 11A. It is to be noted that the capacitor C1 may be electrically coupled between the signal line L1 and the reference potential line Lc, and the resistor R1 may be electrically coupled between the signal line L2 and the reference potential line Lc.

Further, the connection circuit 30 may include the resistor R1 and the capacitor C1 each electrically coupled between the signal line L2 and the reference potential line Lc, as illustrated in FIG. 11B. It is to be noted that the resistor R1 and the capacitor C1 may each be electrically coupled between the signal line L1 and the reference potential line Lc. Configurations of the amplifier circuit 40 and the connection circuit 30 are not limited to the above-described example, and may be changed as appropriate.

### <2-2. Modification Example 2>

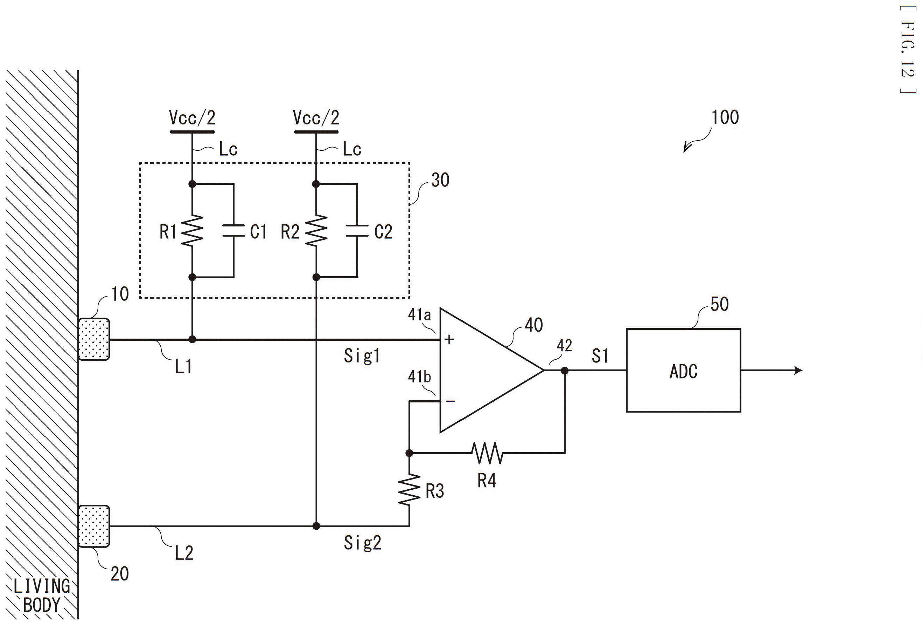

FIG. 12 is a diagram for describing a configuration example of a measurer according to Modification example 2. The amplifier circuit 40 of the measurer 100 may be configured as a non-inverting amplifier circuit or may be configured as an inverting amplifier circuit. In the example illustrated in FIG. 12, the amplifier circuit 40 includes, for example, the input section 41a, the input section 41b, and the output section 42, and includes an amplifier circuit configured to amplify a signal. The amplifier circuit 40 may be configured as, for example, a non-inverting amplifier circuit.

The input section 41a of the amplifier circuit 40 is the first input terminal, and is electrically coupled to the signal line L1. The measurement signal Sig1 is inputted to the input section 41a from the signal electrode 10 via the signal line L1. The input section 41b of the amplifier circuit 40 is the second input terminal. The input section 41b is electrically coupled to the signal line L2 via a resistor R3.

In the example illustrated in FIG. 12, the input section 41a is a positive input terminal, and the input section 41b is a negative input terminal. The output section 42 of the amplifier circuit 40 is an output terminal. A resistor R4 is electrically coupled between the output section 42 and the input section 41b. The amplifier circuit 40 may generate the biological signal S1 having a voltage based on the measurement signal Sig1, with use of the potential of the measurement signal Sig2 as a reference potential, and output the biological signal S1 from the output section 42 to the AD converter circuit 50.

The amplifier circuit 40 may, for example, amplify the measurement signal Sig1 by a predetermined gain (amplification factor), and output an amplified signal. The gain of the amplifier circuit 40 is a value corresponding to a ratio between a resistance value of the resistor R3 and a resistance value of the resistor R4. The biological signal S1 amplified by the amplifier circuit 40 is outputted to the AD converter circuit 50. Also in the biological signal detection device 1 according to the present modification example, it is possible to accurately detect the biological signal by the signal electrode 10 and the reference electrode 20.

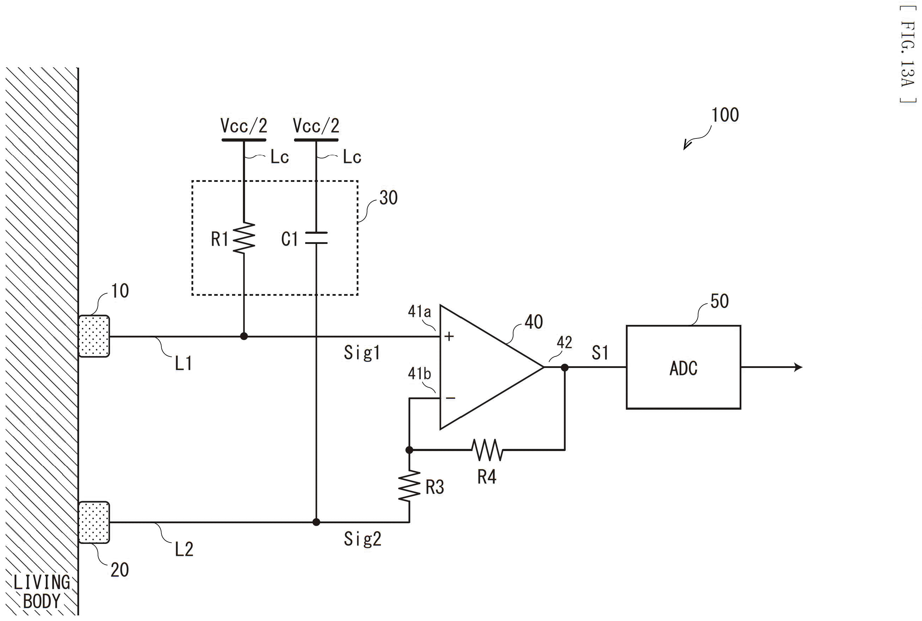

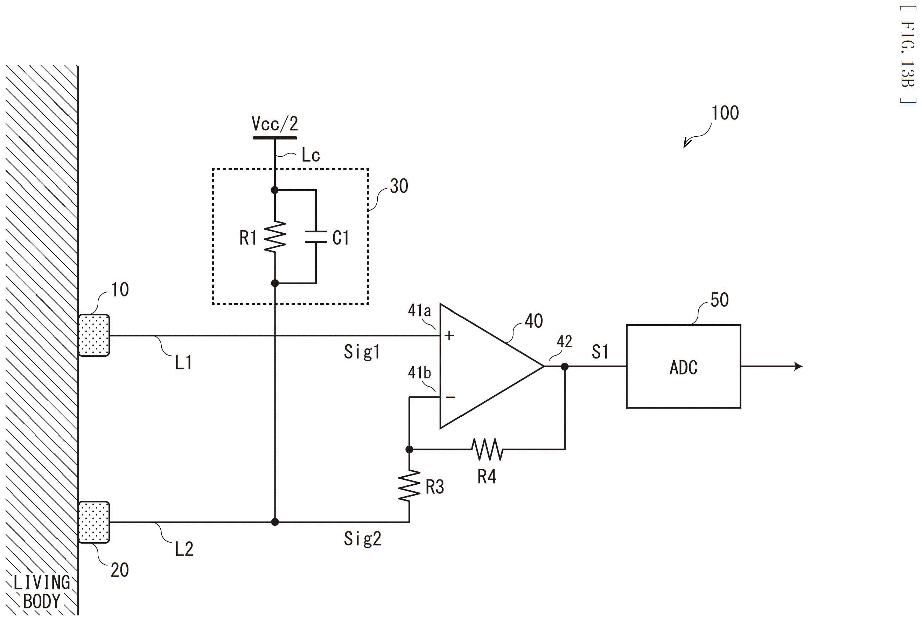

FIGs. 13A and 13B are each a diagram for describing another configuration example of the measurer according to Modification example 2. The configuration of the connection circuit 30 of the measurer 100 is not limited to the above-described example. For example, the connection circuit 30 may include the resistor R1 coupled between the signal line L1 and the reference potential line Lc, and the capacitor C1 coupled between the signal line L2 and the reference potential line Lc, as illustrated in FIG. 13A. It is to be noted that the capacitor C1 may be coupled between the signal line L1 and the reference potential line Lc, and the resistor R1 may be coupled between the signal line L2 and the reference potential line Lc.

As illustrated in FIG. 13B, the connection circuit 30 may include the resistor R1 and the capacitor C1 each coupled between the signal line L2 and the reference potential line Lc. It is to be noted that the resistor R1 and the capacitor C1 may each be coupled between the signal line L1 and the reference potential line Lc.

### <2-3. Modification Example 3>

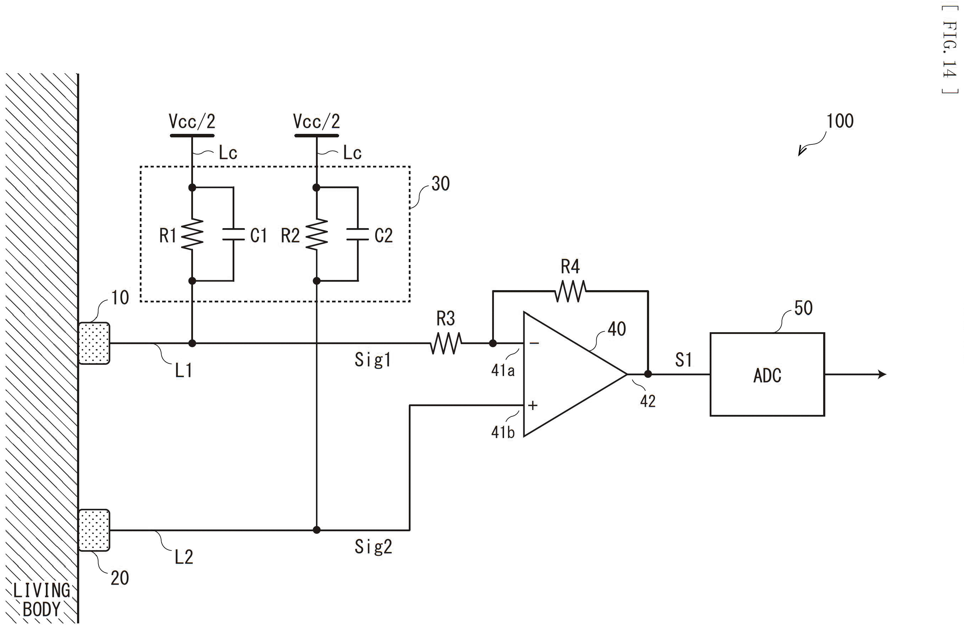

FIG. 14 is a diagram for describing a configuration example of a measurer according to Modification example 3. The amplifier circuit 40 of the measurer 100 may be configured as an inverting amplifier circuit. The amplifier circuit 40 includes the input section 41a, the input section 41b, and the output section 42. In the example illustrated in FIG. 14, the input section 41a is the negative input terminal, and the input section 41b is the positive input terminal.

The input section 41a of the amplifier circuit 40 is electrically coupled to the signal line L1 via the resistor R3. The measurement signal Sig1 is inputted to the input section 41a from the signal electrode 10 via the signal line L1 and the resistor R3. Further, the resistor R4 is electrically coupled between the output section 42 and the input section 41a. The input section 41b of the amplifier circuit 40 is electrically coupled to the signal line L2. The measurement signal Sig2 is inputted to the input section 41b from the reference electrode 20 via the signal line L2.

The amplifier circuit 40 may generate the biological signal S1 based on the potential of the measurement signal Sig1 and the potential of the measurement signal Sig2, and output the biological signal S1 from the output section 42 to the AD converter circuit 50. Also in the biological signal detection device 1 according to the present modification example, it is possible to accurately detect the biological signal.

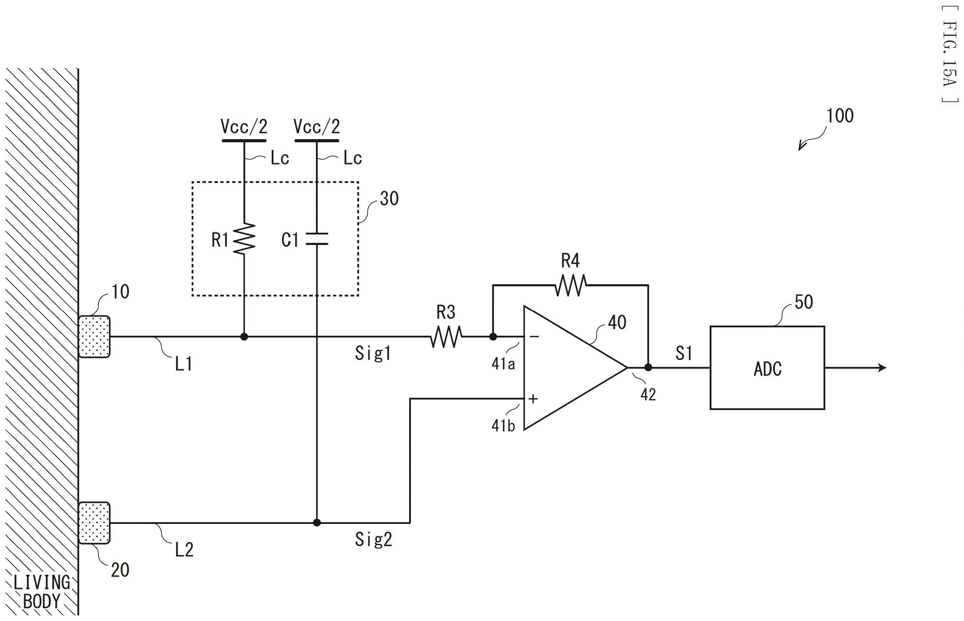

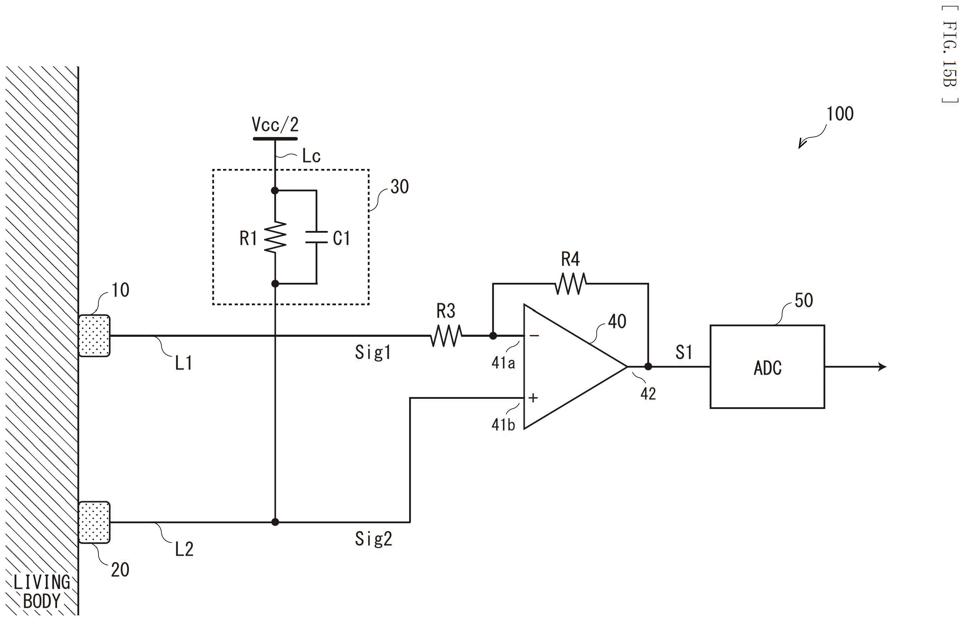

FIGs. 15A and 15B are each a diagram for describing another configuration example of the measurer according to Modification example 3. The configuration of the connection circuit 30 is not limited to the above-described example. For example, the connection circuit 30 may include the resistor R1 that is coupled between the signal line L1 and the reference potential line Lc, and the capacitor C1 that is coupled between the signal line L2 and the reference potential line Lc, as illustrated in FIG. 15A. It is to be noted that the capacitor C1 may be coupled between the signal line L1 and the reference potential line Lc, and the resistor R1 may be coupled between the signal line L2 and the reference potential line Lc.

As illustrated in FIG. 15B, the connection circuit 30 may include the resistor R1 and the capacitor C1 each electrically coupled between the signal line L2 and the reference potential line Lc. It is to be noted that the resistor R1 and the capacitor C1 may each be electrically coupled between the signal line L1 and the reference potential line Lc.

### <2-4. Modification Example 4>

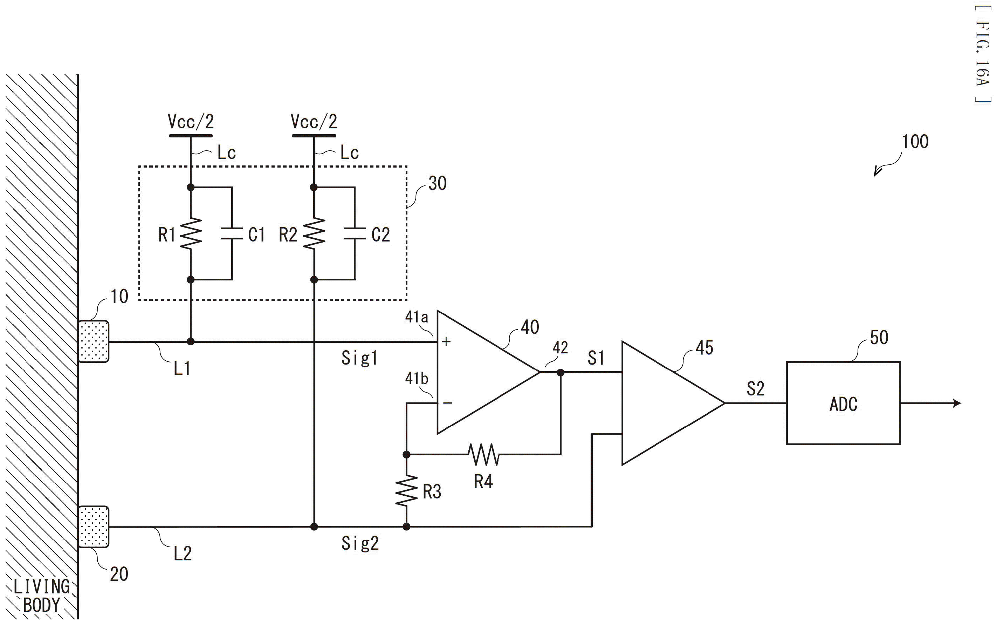

FIGs. 16A to 16C are each a diagram for describing a configuration example of a measurer according to Modification example 4. The measurer 100 may include an amplifier circuit 45, for example, as illustrated in FIG. 16A. The amplifier circuit 45 includes, for example, a differential amplifier circuit (a differential amplifier) configured to amplify a signal.

The amplifier circuit 40 is configured to generate the biological signal S1 based on the measurement signal Sig1 and the measurement signal Sig2, and output the biological signal S1 to the amplifier circuit 45. The amplifier circuit 45 is configured to, for example, generate a biological signal S2 based on the biological signal S1 and the measurement signal Sig2, and output the biological signal S2 to the AD converter circuit 50. The amplifier circuit 45 may generate the biological signal S2 based on a difference between the biological signal S1 and the measurement signal Sig2. The amplifier circuit 45 generates, for example, the biological signal S2 corresponding to a difference between a potential of the biological signal S1 and the potential of the measurement signal Sig2, and outputs the biological signal S2 to the AD converter circuit 50.

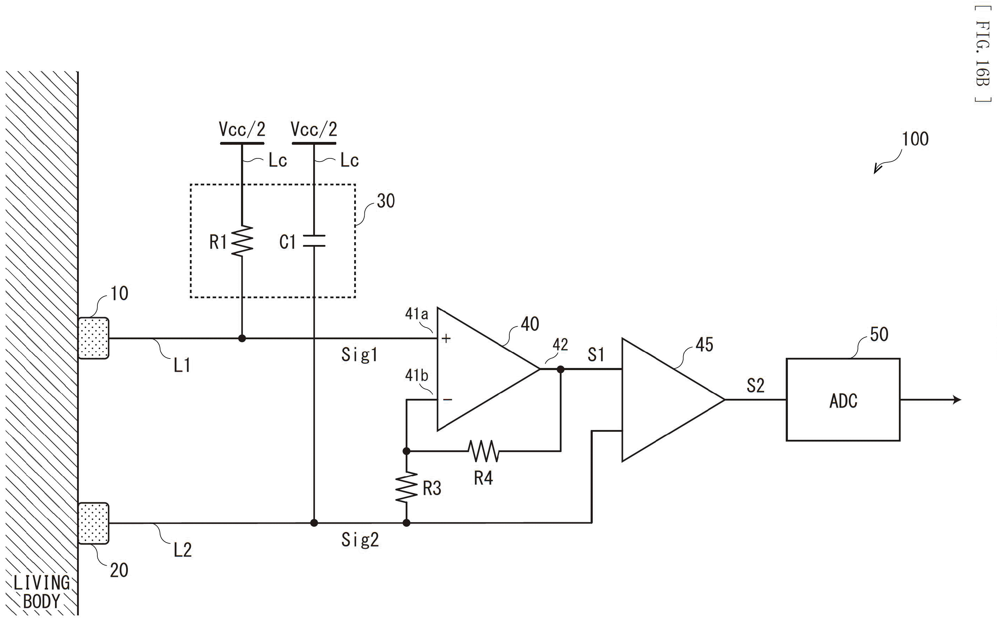

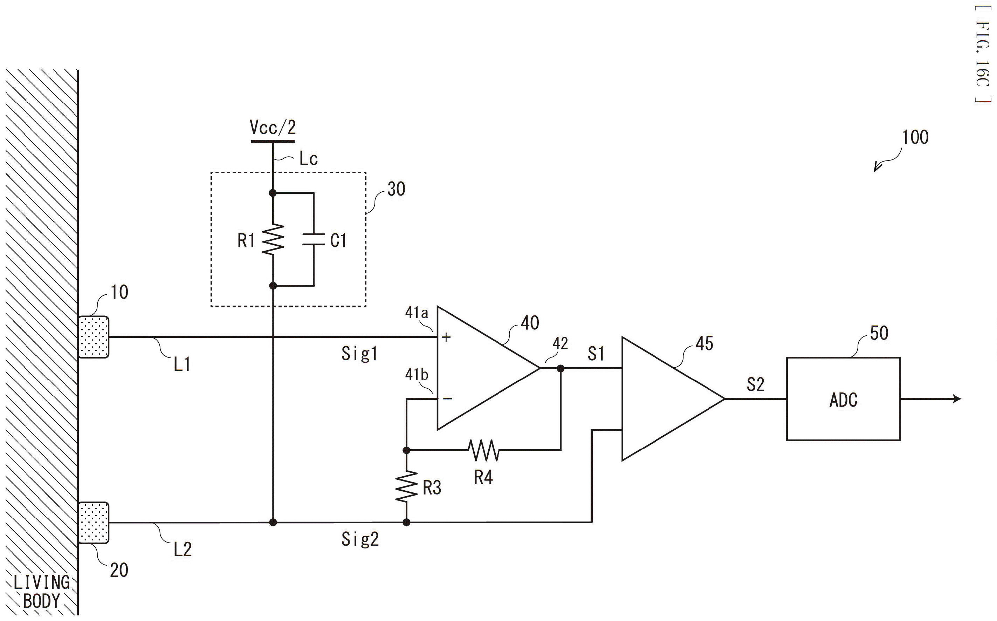

The AD converter circuit 50 converts the biological signal S2 as an analog signal outputted by the amplifier circuit 45 into a digital signal. The AD converter circuit 50 may output the biological signal S2 converted into the digital signal to the signal processor 110 (see FIG. 1). It is to be noted that the configuration of the connection circuit 30 may be changed as appropriate as in the examples illustrated in FIGs. 16A to 16C. The measurer 100 may have the configuration illustrated in FIG. 16B or FIG. 16C.

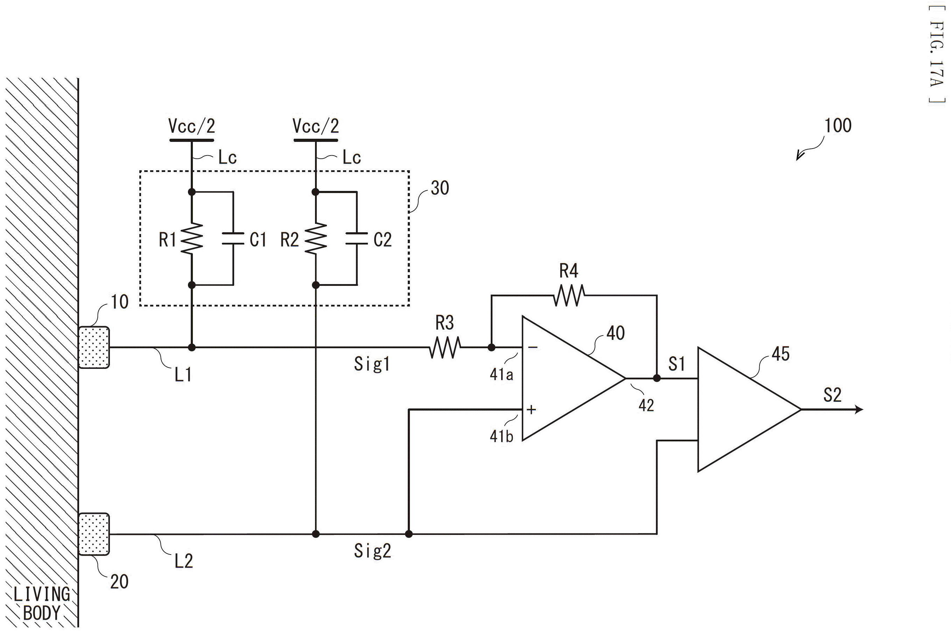

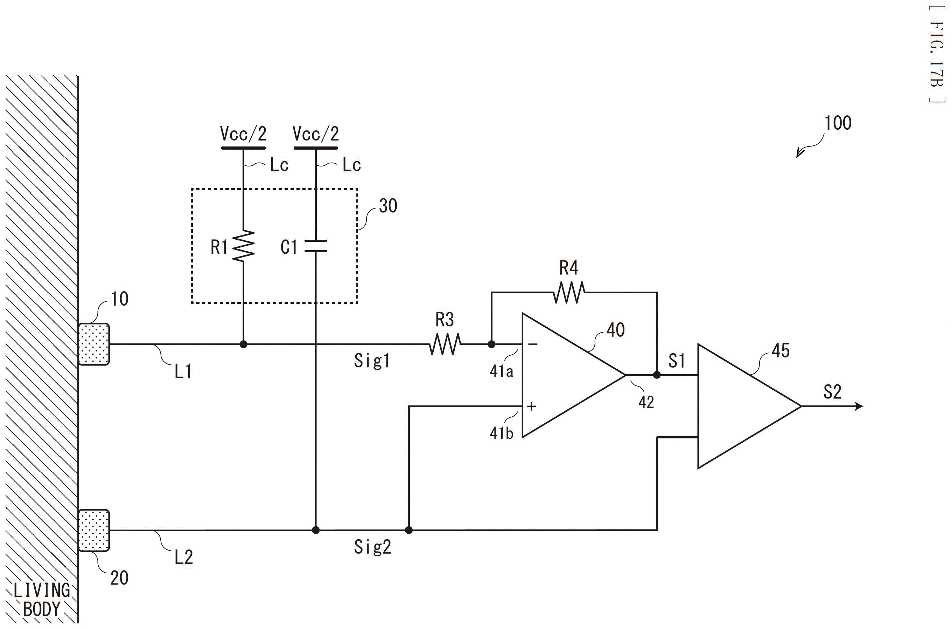

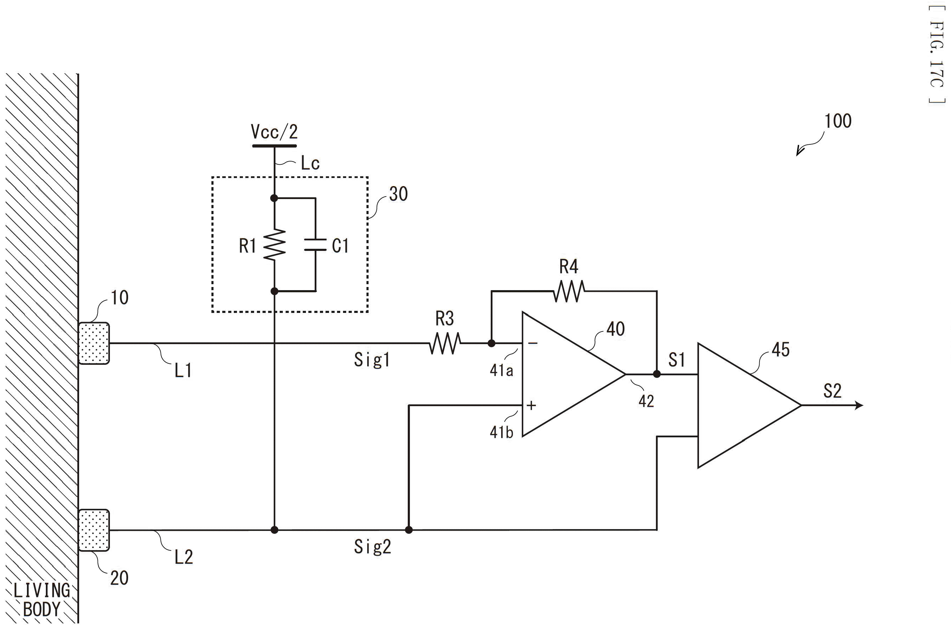

The amplifier circuit 40 may be configured as a non-inverting amplifier circuit or may be configured as an inverting amplifier circuit. The measurer 100 may be configured as illustrated in FIGs. 17A to 17C. The amplifier circuit 45 generates, for example, the biological signal S2 based on the difference between the biological signal S1 and the measurement signal Sig2. The amplifier circuit 45 may generate the biological signal S2 corresponding to the difference between the potential of the biological signal S1 and the potential of the measurement signal Sig2, and output the biological signal S2 to the AD converter circuit 50. It is to be noted that the amplifier circuit 45 may be configured as a differential-output amplifier circuit.

### <2-5. Modification Example 5>

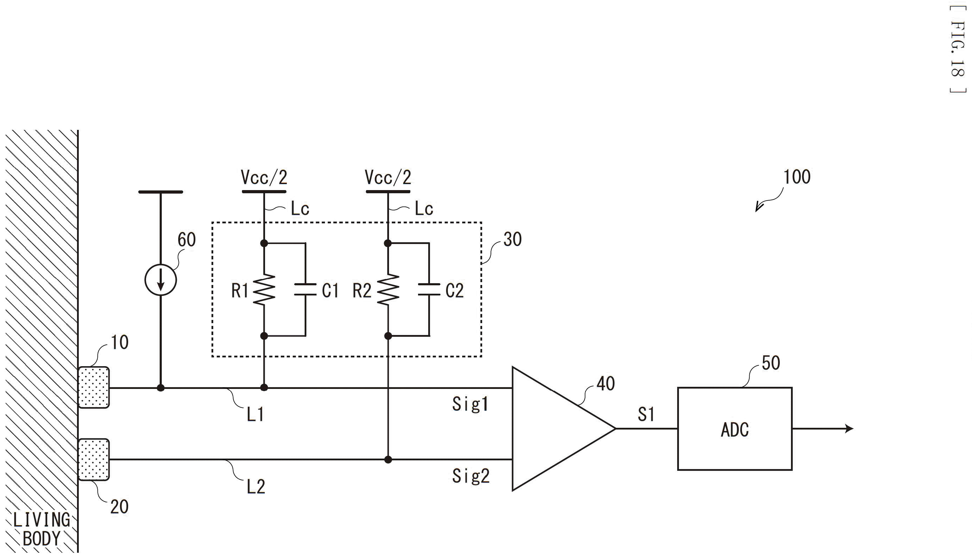

FIG. 18 is a diagram for describing a configuration example of a measurer according to Modification example 5. The measurer 100 may include a current source 60, as illustrated in FIG. 18. The current source 60 is configured to supply a current to the signal line L1 (or the signal line L2). In the example illustrated in FIG. 18, the current source 60 may be electrically coupled to the signal line L1 and may supply the current to the signal line L1. It is to be noted that the current source 60 may be electrically coupled to the signal line L2.

The current source 60 is configured to, for example, supply an alternating current with a specific frequency to the signal line L1. The current source 60 may, for example, supply the alternating current to the signal electrode 10 via the signal line L1. In a case where the signal electrode 10 and the reference electrode 20 are in contact with the living body, the alternating current flows between the signal electrode 10 and the reference electrode 20.

In a case where the current is supplied by the current source 60, the amplifier circuit 40 may generate and output the biological signal S1 corresponding to the potential of the signal electrode 10 and the potential of the reference electrode 20. The biological signal S1 serves as a signal with an amplitude (a signal level) corresponding to an impedance (a contact impedance) between the living body and the electrode. Using the biological signal S1 makes it possible to detect a connection state between the signal electrode 10 and the reference electrode 20. It is possible to determine whether or not the signal electrode 10 (or the reference electrode 20) is in contact with the living body.

It is to be noted that the configurations of the amplifier circuit 40 and the connection circuit 30 are not limited to the example illustrated in FIG. 18, and may be changed as appropriate. For example, the amplifier circuit 40 may be a single-output amplifier circuit or may be a differential-output amplifier circuit. The amplifier circuit 40 may be configured as a non-inverting amplifier circuit or may be configured as an inverting amplifier circuit.

### <2-6. Modification Example 6>

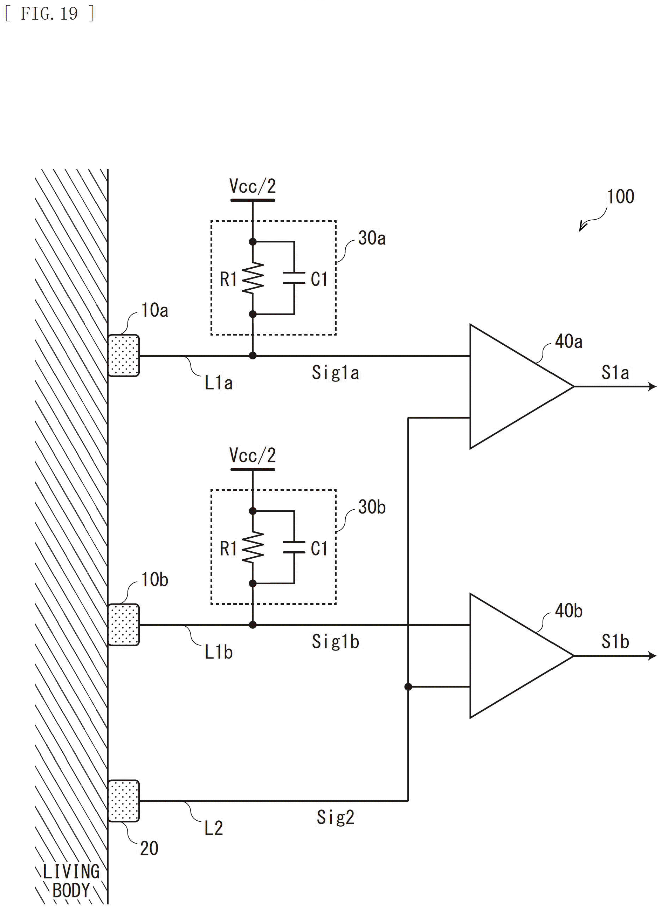

FIG. 19 is a diagram for describing a configuration example of a measurer according to Modification example 6. Described in the above-described embodiment is an example in which the measurer 100 includes one signal electrode 10 and one reference electrode 20, but the signal electrode 10 and the reference electrode 20 are each not limited to such number and arrangement. The measurer 100 of the biological signal detection device 1 may, for example, include a plurality of signal electrodes 10, using the reference electrode 20 as a common electrode.

The biological signal detection device 1 may include, for example, pairs (sets) of the signal electrode 10 and the reference electrode 20, and may be configured to detect a plurality of biological signals. The measurer 100 may include two or more pairs and may be configured to acquire two or more (two or more channels of) signals, as in the example illustrated in FIG. 19. The measurer 100 may, for example, include the amplifier circuit 40 for each signal electrode 10.

In the example illustrated in FIG. 19, the measurer 100 includes a signal electrode 10a, a signal electrode 10b, the reference electrode 20, a connection circuit 30a, a connection circuit 30b, an amplifier circuit 40a, and an amplifier circuit 40b. For example, the connection circuit 30a is provided for a signal line L1a coupled to the signal electrode 10a, and the connection circuit 30b is provided for a signal line L1b coupled to the signal electrode 10b.

The amplifier circuit 40a is configured to generate, for example, the biological signal S1a based on a measurement signal Sig1a corresponding to a potential of the signal electrode 10a and the measurement signal Sig2. The amplifier circuit 40a may, for example, generate the biological signal S1a corresponding to a difference between the potential of the measurement signal Sig1a and the potential of the measurement signal Sig2, and output the biological signal S1a to the AD converter circuit 50.

The amplifier circuit 40b is configured to generate, for example, the biological signal S1b based on a measurement signal Sig1b corresponding to a potential of the signal electrode 10b and the measurement signal Sig2. The amplifier circuit 40b may, for example, generate the biological signal S1b corresponding to a difference between the potential of the measurement signal Sig1b and the potential of the measurement signal Sig2, and output the biological signal S1b to the AD converter circuit 50.

It is to be noted that the biological signal detection device 1 may be configured to detect three or more (three or more channels of) biological signals. The biological signal detection device 1 may be configured to detect biological signals at three or more sites. It is to be noted that configurations of a plurality of amplifier circuits 40 (the amplifier circuits 40a and 40b in FIG. 19) and a plurality of connection circuits 30 (the connection circuits 30a and 30b in FIG. 19) are not limited to the illustrated example, and may be changed as appropriate.

### <3. Second Embodiment>

Next, description is given of a second embodiment of the present disclosure. Hereinafter, components similar to those in the foregoing embodiment are denoted by the same reference numerals, and descriptions thereof are omitted as appropriate.

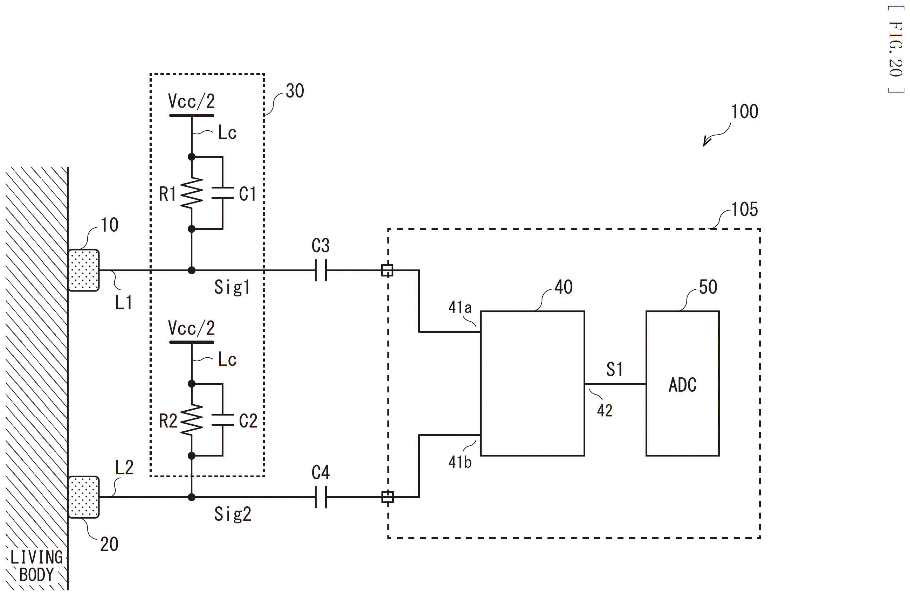

FIG. 20 is a diagram illustrating a configuration example of the measurer according to the second embodiment of the present disclosure. The measurer 100 includes a capacitor C3 and a capacitor C4, as in the example illustrated in FIG. 20. Each of the capacitor C3 and the capacitor C4 is a capacitive impedance element, and is a capacitor (a condenser).

The measurer 100 also includes a semiconductor chip 105 including the amplifier circuit 40 and the AD converter circuit 50. The semiconductor chip 105 includes, for example, a semiconductor substrate including an analog circuit, and may be referred to as an AFE (Analog Front End) chip. It is to be noted that the semiconductor chip 105 may be provided with the signal processor 110 and the estimator 120 described above.

The capacitor C3 is coupled between the signal electrode 10 and the amplifier circuit 40. The capacitor C4 is coupled between the reference electrode 20 and the amplifier circuit 40. The capacitor C3 and the capacitor C4 may each be a passive element or may each include an active element. The capacitors C3 and C4 may each include a capacitor such as a MOS capacitance or a MIM capacitance. The capacitors C3 and C4 may each include a variable capacitor.

The capacitor C3 is electrically coupled in series between the signal line L1 electrically coupled to the signal electrode 10, and the input section 41a of the amplifier circuit 40, as in the example illustrated in FIG. 20. In the example illustrated in FIG. 20, one electrode (terminal) of the capacitor C3 is electrically coupled to the signal electrode 10, the resistor R1, and the capacitor C1. Further, the other electrode of the capacitor C3 is electrically coupled to the input section 41a of the amplifier circuit 40.

The capacitor C4 is electrically coupled in series between the signal line L2 electrically coupled to the reference electrode 20, and the input section 41b of the amplifier circuit 40. In the example illustrated in FIG. 20, one electrode of the capacitor C4 is electrically coupled to the reference electrode 20, the resistor R2, and the capacitor C2. Further, the other electrode of the capacitor C4 is electrically coupled to the input section 41b of the amplifier circuit 40.

To the input section 41a of the amplifier circuit 40, the measurement signal Sig1 corresponding to the potential of the signal electrode 10 is inputted via the signal line L1 and the capacitor C3. Further, to the input section 41b of the amplifier circuit 40, the measurement signal Sig2 corresponding to the potential of the reference electrode 20 is inputted via the signal line L2 and the capacitor C4.

The amplifier circuit 40 is configured to output the biological signal S1 based on the measurement signal Sig1 and the measurement signal Sig2. The amplifier circuit 40 may, for example, generate the biological signal S1 corresponding to the potential of the measurement signal Sig1 and the potential of the measurement signal Sig2, and output the biological signal S1 from the output section 42 to the AD converter circuit 50.

The biological signal detection device 1 according to the present embodiment includes the capacitor C3 and the capacitor C4 as described above. This enables the biological signal detection device 1 to obtain the measurement signals Sig1 and Sig2 from which DC components (direct-current components) has been removed. It is possible to accurately detect the biological signal using the measurement signals resulting from cutting the DC components.

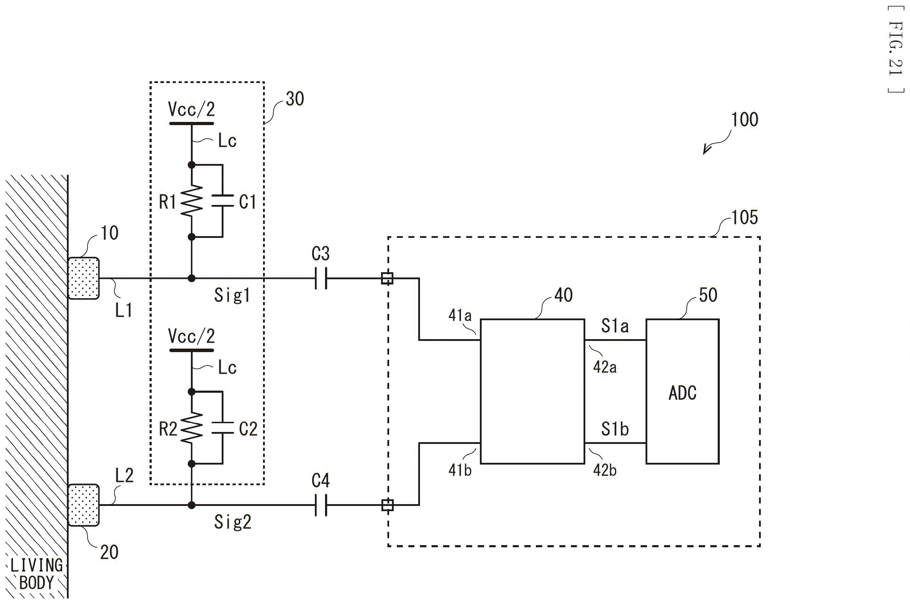

It is to be noted that the amplifier circuit 40 of the measurer 100 may be a single-output amplifier circuit or may be a differential-output amplifier circuit. For example, the amplifier circuit 40 may be configured as a differential-output amplifier circuit, as in the example illustrated in FIG. 21. The amplifier circuit 40 may be configured to output the biological signal S1a and the biological signal S1b as the inverted signal of the biological signal S1a.

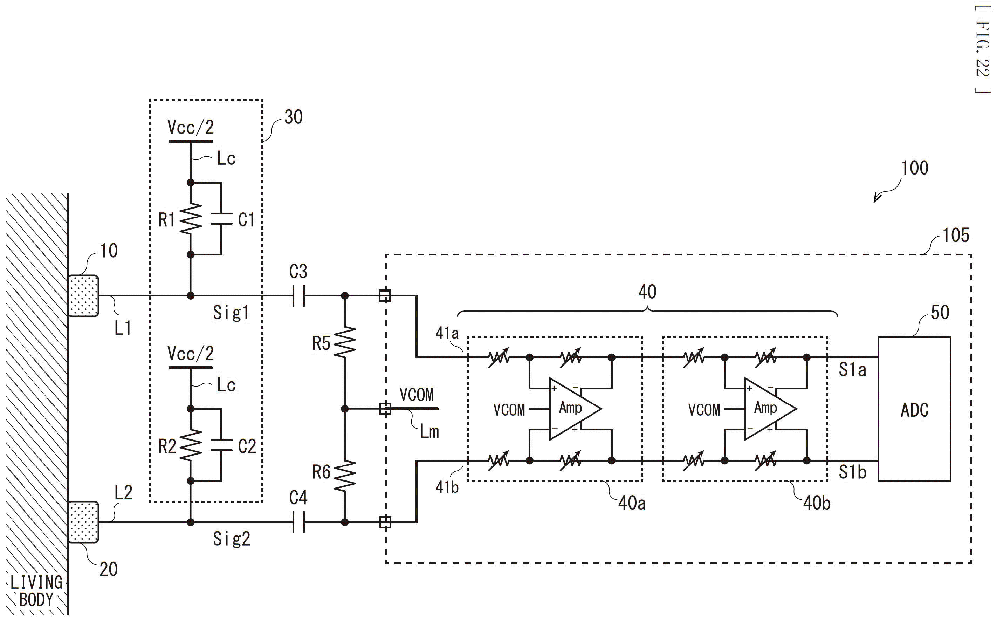

FIG. 22 is a diagram for describing a configuration example of the measurer according to the second embodiment. The amplifier circuit 40 includes, for example, a single-stage amplifier circuit or a plurality of stages of amplifier circuits. For example, the amplifier circuit 40 may include a plurality of stages of amplifier circuits (the amplifier circuit 40a and the amplifier circuit 40b in the example illustrated in FIG. 22).

The amplifier circuit 40a and the amplifier circuit 40b each include, for example, a differential amplifier circuit. The amplifier circuits 40a and 40b include, for example, an instrumentation amplifier. It is to be noted that the configuration of the amplifier circuit 40 is not limited to the illustrated example, and may be changed as appropriate. The amplifier circuit 40 may include a single-stage amplifier circuit, for example, only one of the amplifier circuits 40a and 40b.

The measurer 100 may include a resistor R5 and a resistor R6, as illustrated in FIG. 22. The resistor R5 and the resistor R6 are resistive impedance elements. The resistors R5 and R6 may each be a passive element or may each include an active element. The resistors R5 and R6 may each include a variable resistor.

The resistor R5 is electrically coupled between the input section 41a of the amplifier circuit 40 and a reference potential line Lm, as in the example illustrated in FIG. 22. The resistor R6 is electrically coupled between the input section 41b of the amplifier circuit 40 and the reference potential line Lm. The reference potential line Lm is a wiring to which a predetermined voltage VCOM is applied.

The reference potential line Lm is, for example, a wiring to which a potential (an intermediate potential) between a potential of a power supply line and a ground potential is applied. For example, the reference potential line Lm is supplied with a voltage that is half the power supply voltage Vcc, that is, Vcc/2, as the voltage VCOM. The reference potential line Lm serves as a potential line to which a potential corresponding to the potential of the power supply line of the measurer 100 is applied. It is to be noted that the reference potential line Lc and the reference potential line Lm may be set to the same voltage (voltage value), for example, Vcc/2.

In the example illustrated in FIG. 22, one end (one terminal) of the resistor R5 is electrically coupled to the capacitor C3 and the input section 41a. The other end (the other terminal) of the resistor R5 is electrically coupled to the reference potential line Lm. One end of the resistor R6 is electrically coupled to the capacitor C4 and the input section 41b. The other end of the resistor R6 is electrically coupled to the reference potential line Lm.

The reference potential line Lm is electrically coupled to the capacitor C3 and the input section 41a via the resistor R5. Further, the reference potential line Lm is electrically coupled to the capacitor C4 and the input section 41b via the resistor R6. It is to be noted that the reference potential line Lm to which the voltage VCOM is applied may be electrically coupled to the amplifier circuit 40a and the amplifier circuit 40b, as illustrated in FIG. 22.

Capacitance values of the capacitors C3 and C4 and resistance values of the resistors R5 and R6 are set on the basis of, for example, the frequency of the signal that is inputted to the signal line L1 (or the signal line L2). The measurer 100 has a cut-off frequency based on the capacitance value of the capacitor C3 (or the capacitor C4) and the resistance value of the resistor R5 (or the resistor R6).

For example, in a case of setting the cut-off frequency to about 0.1 Hz in the example illustrated in FIG. 22, capacitor C3 = 15 nF, capacitor C4 = 15 nF, resistor R5 = 100 MΩ, and resistor R6 = 100 MΩ may be set. The capacitance values of the capacitors C3 and C4 and the resistance values of the resistors R5 and R6 may be set as appropriate depending on the frequency of the signal to be measured.

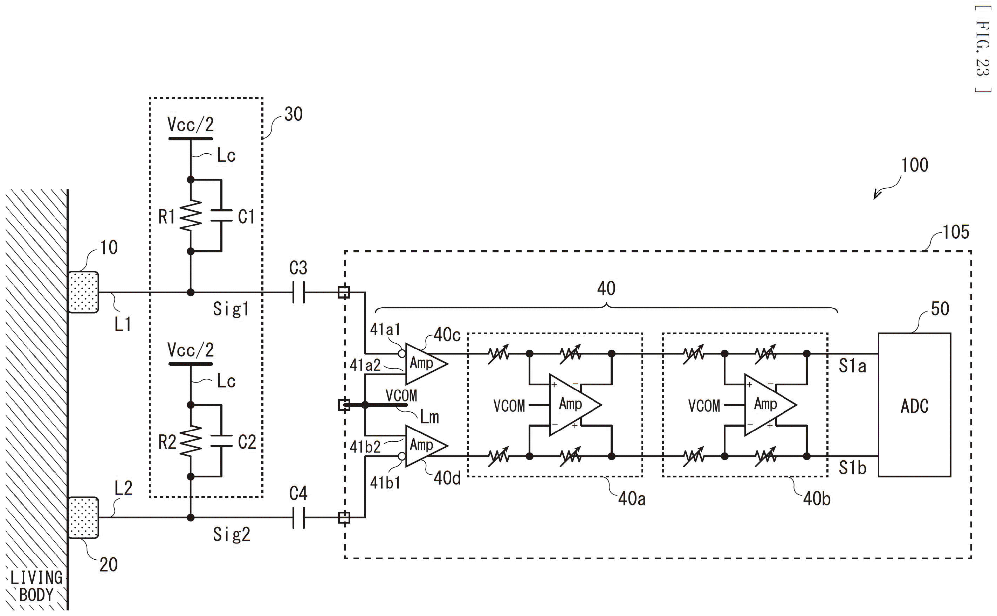

FIG. 23 is a diagram for describing another configuration example of the measurer according to the second embodiment. The measurer 100 may include an amplifier circuit 40c and an amplifier circuit 40d, as in the example illustrated in FIG. 23. For example, the amplifier circuit 40c and the amplifier circuit 40d include differential amplifier circuits and are provided on the semiconductor chip 105. The amplifier circuit 40 may include the amplifier circuit 40c and the amplifier circuit 40d.

The amplifier circuit 40c includes, for example, an input section 41a1 and an input section 41a2, as illustrated in FIG. 23, and includes an amplifier circuit configured to amplify a signal. The input section 41a1 of the amplifier circuit 40c is the first input terminal, and is electrically coupled to the capacitor C3. The measurement signal Sig1 is inputted to the input section 41a1 from the signal electrode 10 via the signal line L1 and the capacitor C3.

The input section 41a2 of the amplifier circuit 40c is the second input terminal, and is electrically coupled to the reference potential line Lm. The voltage VCOM is applied to the input section 41a2 by the reference potential line Lm. The amplifier circuit 40c may output a signal based on a difference between the measurement signal Sig1 and the voltage VCOM to the amplifier circuit 40a.

The amplifier circuit 40d includes, for example, an input section 41b1 and an input section 41b2, and includes an amplifier circuit configured to amplify a signal. The input section 41b1 of the amplifier circuit 40d is the first input terminal, and is electrically coupled to the capacitor C4. The measurement signal Sig2 is inputted to the input section 41b1 from the reference electrode 20 via the signal line L2 and the capacitor C4.

The input section 41b2 of the amplifier circuit 40d is the second input-terminal, and is electrically coupled to the reference potential line Lm. The voltage VCOM is applied to the input section 41b2 by the reference potential line Lm. The amplifier circuit 40d may output a signal based on a difference between the measurement signal Sig2 and the voltage VCOM to the amplifier circuit 40a.

The amplifier circuit 40a and the amplifier circuit 40b are configured to, for example, output the biological signal S1a and the biological signal S1b, with use of an output signal of the amplifier circuit 40c and an output signal of the amplifier circuit 40d as differential input signals. The amplifier circuit 40 may generate the biological signals S1a and S1b by the amplifier circuits 40a and 40b, and output the biological signals S1a and S1b to the AD converter circuit 50.

The biological signal detection device 1 includes the amplifier circuit 40c and the amplifier circuit 40d as described above. The input section 41a1 and the input section 41a2 of the amplifier circuit 40c are in a virtual short state, and the input section 41b1 and the input section 41b2 of the amplifier circuit 40d are in a virtual short state. This allows the measurer 100 to have a configuration without the resistors R5 and R6 described above, and makes it possible to increase an input impedance as seen from the capacitor C3 and the capacitor C4. This makes it possible to reduce the capacitance values of the capacitors C3 and C4.

As compared with a case where the biological signal detection device 1 does not include the amplifier circuits 40c and 40d, it is possible to make the capacitance values of the capacitors C3 and C4 smaller, making it possible to prevent the capacitors C3 and C4 from increasing in size. For example, it is possible to make the values of the capacitors C3 and C4 smaller than those in the example illustrated in FIG. 22.

In the example illustrated in FIG. 23, in a case of setting the cut-off frequency of a DC component removing filter including the capacitor C3 and the amplifier circuit 40c to about 0.1 Hz, for example, it is possible to set capacitor C3 = 100 pF, assuming that the input impedance formed by the amplifier circuit 40c is 1 GΩ. Similarly, it is possible to set capacitor C4 = 100 pF.

### <Workings and Effects>

The biological signal detection device (the biological signal detection device 1) according to the present embodiment includes: a first amplifier circuit (e.g., the amplifier circuit 40) including a first input section (e.g., the input section 41a) and a second input section (the input section 41b); a third capacitor (the capacitor C3) that is electrically coupled in series between the first signal line (e.g., the signal line L1) and the first input section; and a fourth capacitor (the capacitor C4) that is electrically coupled in series between the second signal line (the signal line L2) and the second input section.

In the biological signal detection device 1 according to the present embodiment, the capacitor C3 that is electrically coupled in series between the signal line L1 and the input section 41a, and the capacitor C4 that is electrically coupled in series between the signal line L2 and the input section 41b are provided. This makes it possible to accurately detect the biological signal. It is possible to achieve a biological signal detection device having favorable detection performance.

### <4. Modification Examples>

### <4-1. Modification Example 7>

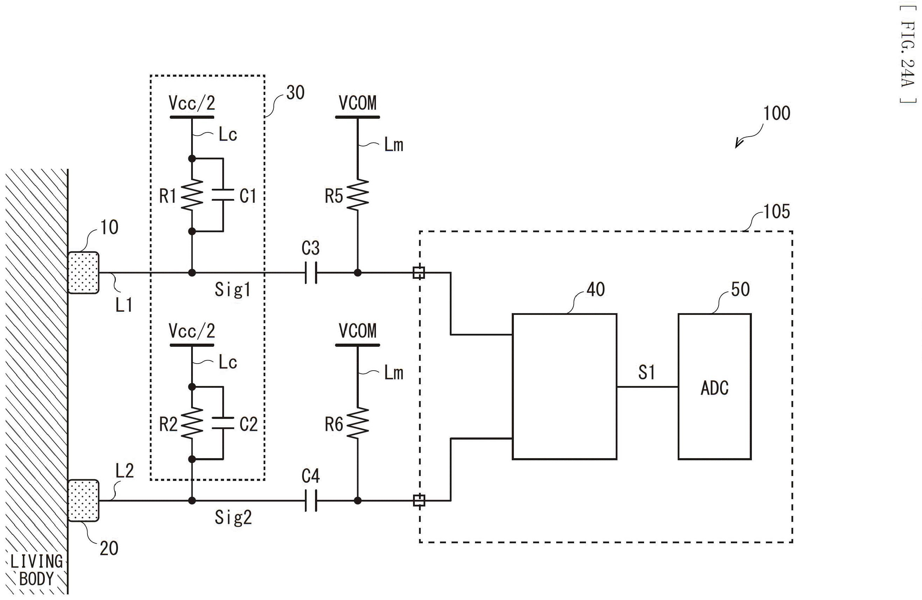

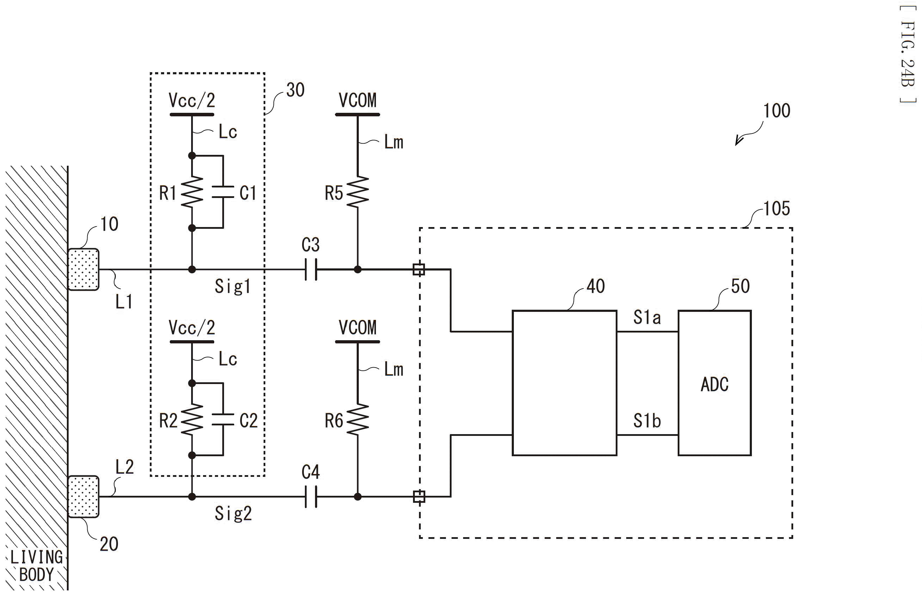

Although configuration examples of the biological signal detection device 1 have been described in the above-described embodiment, the configuration is merely an example. The configuration of the biological signal detection device 1 is not limited to the illustrated example, and may be changed as appropriate. FIGs. 24A and 24B are each a diagram for describing a configuration example of the measurer according to Modification example 7 of the present disclosure. The amplifier circuit 40 of the measurer 100 may be configured as a single-output amplifier circuit or may be configured as a differential-output amplifier circuit.

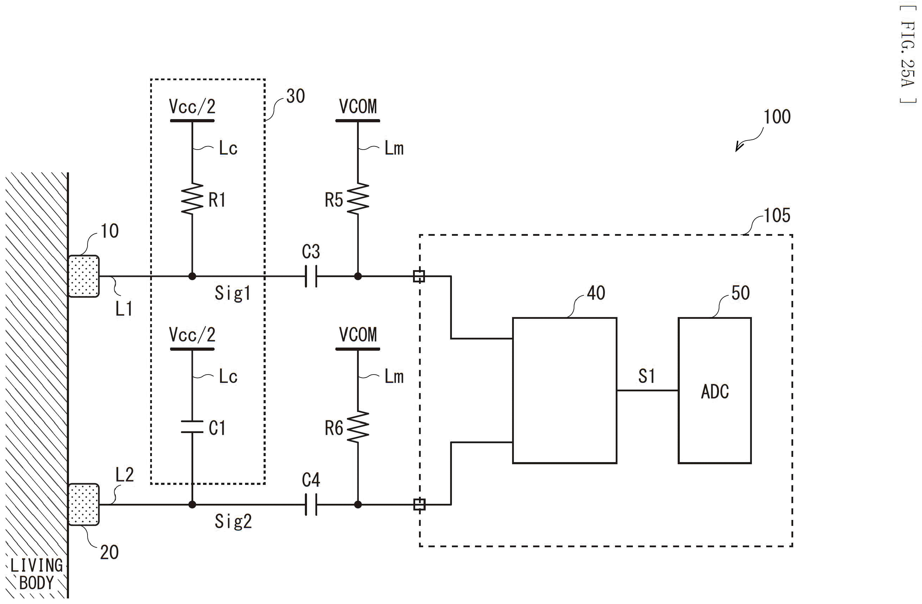

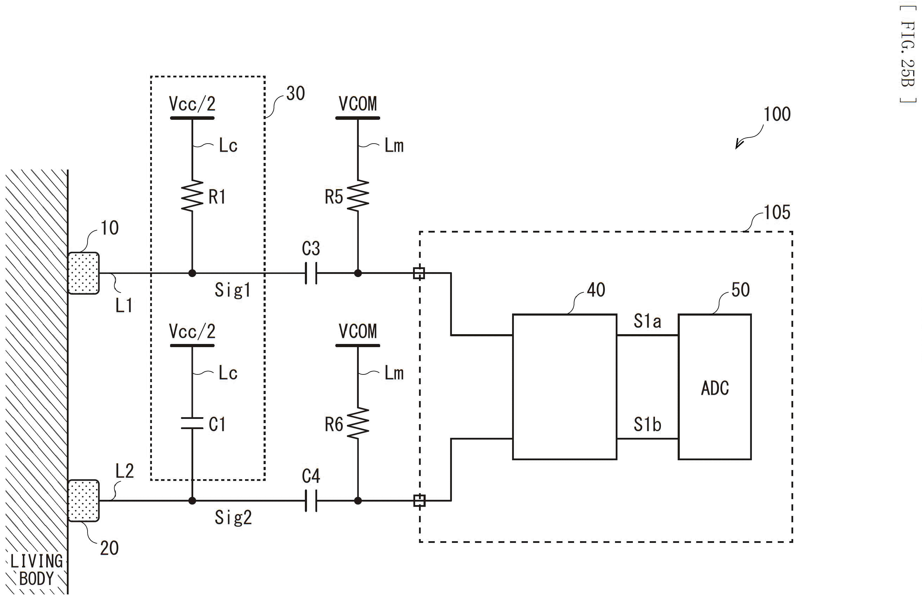

FIGs. 25A and 25B are each a diagram for describing another configuration example of the measurer according to Modification example 7. The connection circuit 30 may include the resistor R1 electrically coupled between the signal line L1 and the reference potential line Lc, and the capacitor C1 electrically coupled between the signal line L2 and the reference potential line Lc. The capacitor C1 may be electrically coupled between the signal line L1 and the reference potential line Lc, and the resistor R1 may be electrically coupled between the signal line L2 and the reference potential line Lc.

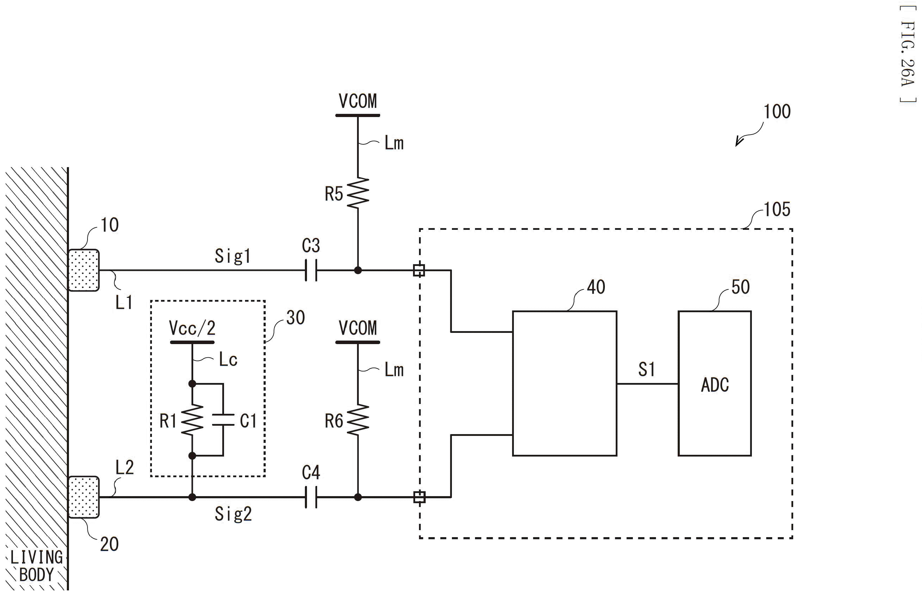

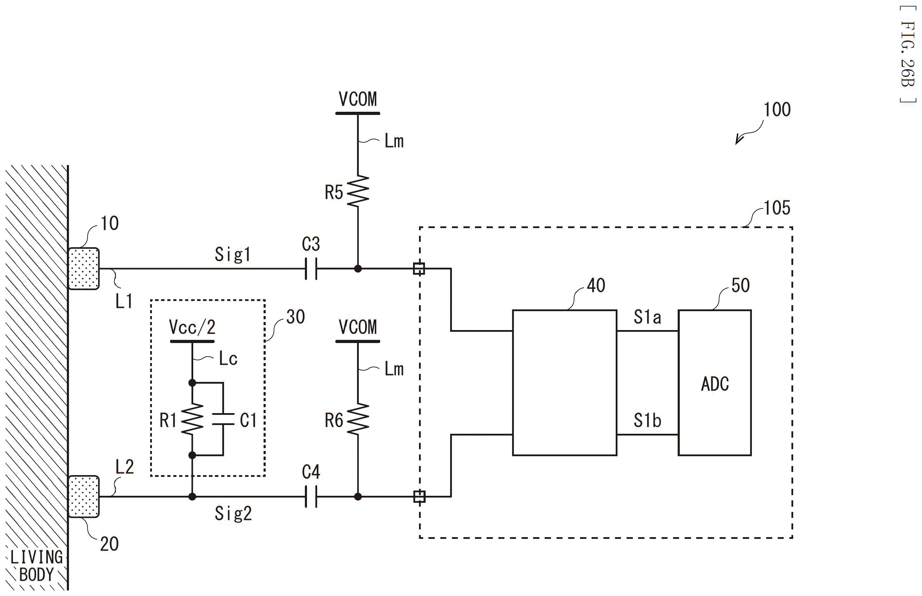

FIGs. 26A and 26B are each a diagram for describing another configuration example of the measurer according to Modification example 7. As in the example illustrated in FIG. 26A or FIG. 26B, the connection circuit 30 may include the resistor R1 and the capacitor C1 each electrically coupled between the signal line L2 and the reference potential line Lc. The resistor R1 and the capacitor C1 may each be electrically coupled in parallel to each other between the signal line L1 and the reference potential line Lc.

It is to be noted that the amplifier circuit 40 of the measurer 100 may include a plurality of stages of amplifier circuits, for example, the amplifier circuit 40a and the amplifier circuit 40b, as described above with reference to FIGs. 22, 23, etc. At least one of the amplifier circuits 40a and 40b, for example, the amplifier circuit 40a, may be configured as a non-inverting amplifier circuit or may be configured as an inverting amplifier circuit, as in the examples illustrated in FIGs. 12, 14, etc. The amplifier circuit 40 may include a single-stage amplifier circuit.

Further, the amplifier circuit 40 may include the amplifier circuit 40c and the amplifier circuit 40d, as described above with reference to FIG. 23. For example, the amplifier circuit 40 including the amplifier circuits 40c and 40d may be provided on the semiconductor chip 105. Configuring the measurer 100 to include the amplifier circuits 40c and 40d makes it possible to omit the resistors R5 and R6.

### <4-2. Modification Example 8>

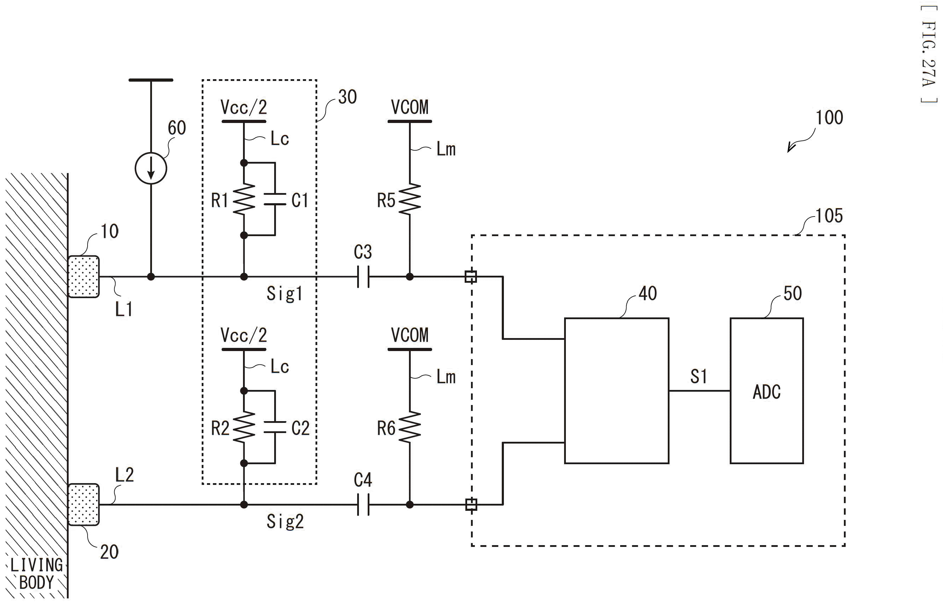

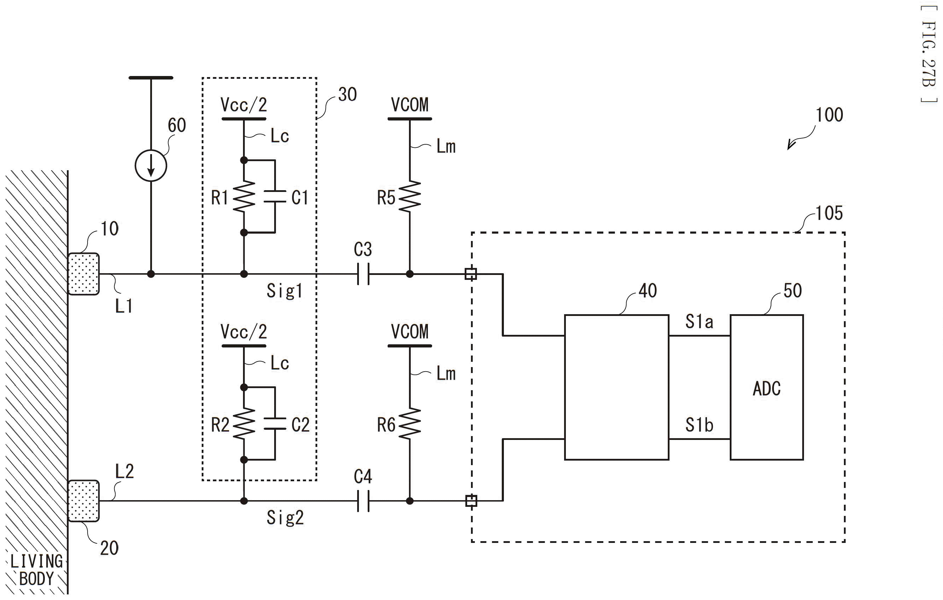

FIGs. 27A and 27B are each a diagram for describing a configuration example of the measurer according to Modification example 8. The measurer 100 may include the current source 60 as described above, as in the example illustrated in FIG. 27A or FIG. 27B. The current source 60 is configured to supply a current to the signal line L1 (or the signal line L2). The current source 60 may be, for example, electrically coupled to the signal line L1 and may supply the current to the signal line L1. It is to be noted that the current source 60 may be electrically coupled to the signal line L2.

Although the description has been given hereinabove of the present disclosure with reference to the embodiments and modification examples, the present technology is not limited to the foregoing embodiments and the like, and may be modified in a wide variety of ways. For example, although the above-described modification examples have been described as the modification examples of the above-described embodiments, the configurations of the respective modification examples may be combined as appropriate. In addition, the present disclosure has applicability not only to the human body, but also to a living body other than the human body, for example, animals such as pets and domestic animals.

A biological signal detection device according to an embodiment of the present disclosure includes: a first electrode and a second electrode that are configured to be in contact with a living body; a first signal line that is electrically coupled to the first electrode; a second signal line that is electrically coupled to the second electrode; a first resistor that is electrically coupled between the first signal line or the second signal line and a first potential line; and a first capacitor that is electrically coupled between the first signal line or the second signal line and the first potential line. Accordingly, it is possible to achieve the biological signal detection device that makes it possible to reduce the number of electrodes.

It is to be noted that the effects described in the present specification are merely examples and are not limited to the description, and other effects may be obtained. Further, the present disclosure may have the following configuration.
(1) A biological signal detection device including:
   a first electrode and a second electrode that are configured to be in contact with a living body;
   a first signal line that is electrically coupled to the first electrode;
   a second signal line that is electrically coupled to the second electrode;
   a first resistor that is electrically coupled between the first signal line or the second signal line and a first potential line; and
   a first capacitor that is electrically coupled between the first signal line or the second signal line and the first potential line.
(2) The biological signal detection device according to (1), further including a measurement circuit that includes the first electrode and the second electrode and is configured to generate a biological signal, in which
   the measurement circuit is configured to generate one signal as the biological signal with use of only the first electrode and the second electrode as electrodes configured to be in contact with the living body.
(3) The biological signal detection device according to (2), in which
   the measurement circuit includes a first amplifier circuit that is electrically coupled to the first signal line and the second signal line, and
   the first amplifier circuit is configured to output, as the biological signal, a first signal based on a potential of the first signal line and a potential of the second signal line.
(4) The biological signal detection device according to any one of (1) to (3), in which a resistance value of the first resistor and a capacitance value of the first capacitor are set on the basis of a frequency of a signal that is inputted to the first signal line or the second signal line.
(5) The biological signal detection device according to any one of (1) to (4), in which a cut-off frequency based on a resistance value of the first resistor and a capacitance value of the first capacitor is set on the basis of a frequency to be transmitted of a signal that is inputted to the first signal line or the second signal line.
(6) The biological signal detection device according to any one of (1) to (5), in which a resistance value of the first resistor and a capacitance value of the first capacitor are determined on the basis of a frequency to be acquired of a brainwave.
(7) The biological signal detection device according to any one of (1) to (6), further including a first amplifier circuit configured to output a first signal based on a potential of the first signal line and a potential of the second signal line.
(8) The biological signal detection device according to (7), in which only the first electrode and the second electrode are provided, as electrodes configured to be in contact with the living body, for the first amplifier circuit.
(9) The biological signal detection device according to (7) or (8), in which the first amplifier circuit is configured to output the first signal based on differential amplification between the potential of the first signal line and the potential of the second signal line.
(10) The biological signal detection device according to any one of (7) to (9), in which
   the first amplifier circuit is electrically coupled to a power supply line, and
   the first potential line includes a wiring to which a potential between a potential of the power supply line and a ground potential is applied.
(11) The biological signal detection device according to any one of (7) to (9), in which
   the first amplifier circuit is electrically coupled to a power supply line, and
   the first potential line includes a wiring to which a ground potential is applied.
(12) The biological signal detection device according to any one of (1) to (11), in which the first resistor and the first capacitor are each electrically coupled between the first signal line and the first potential line.
(13) The biological signal detection device according to any one of (1) to (12), in which the first resistor and the first capacitor are electrically coupled in parallel to each other between the first signal line and the first potential line.
(14) The biological signal detection device according to (13), further including a second resistor and a second capacitor that are electrically coupled in parallel to each other between the second signal line and the first potential line.
(15) The biological signal detection device according to any one of (1) to (11), in which
   the first resistor is electrically coupled in series between the first signal line and the first potential line, and
   the first capacitor is electrically coupled in series between the second signal line and the first potential line.
(16) The biological signal detection device according to any one of (1) to (15), further including a first amplifier circuit including a first input section that is electrically coupled to the first electrode via the first signal line, a second input section that is electrically coupled to the second electrode via the second signal line, and an output section, in which
   the first amplifier circuit is configured to output a first signal based on a potential of the first signal line and a potential of the second signal line from the output section.
(17) The biological signal detection device according to (16), in which the first amplifier circuit is configured to output a second signal that is an inverted signal of the first signal.
(18) The biological signal detection device according to (16) or (17), further including:
   a third resistor that is electrically coupled between the second signal line and the second input section; and
   a fourth resistor that is electrically coupled between the second input section and the output section.
(19) The biological signal detection device according to any one of (16) to (18), further including a second amplifier circuit configured to output a second signal based on the first signal and the potential of the second signal line.
(20) The biological signal detection device according to any one of (1) to (19), further including a current source configured to supply a current to the first signal line.
(21) The biological signal detection device according to (20), in which the current source is configured to supply an alternating current with a specific frequency to the first signal line.
(22) The biological signal detection device according to any one of (1) to (21), further including:
   a third electrode configured to be in contact with the living body;
   a third signal line that is electrically coupled to the third electrode;
   a first amplifier circuit that is electrically coupled to the first signal line and the second signal line, and is configured to output a signal based on a potential of the first signal line and a potential of the second signal line; and
   a third amplifier circuit that is electrically coupled to the second signal line and the third signal line, and is configured to output a signal based on the potential of the second signal line and a potential of the third signal line.
(23) The biological signal detection device according to any one of (1) to (22), further including
   a first amplifier circuit including a first input section and a second input section;
   a third capacitor that is electrically coupled in series between the first signal line and the first input section; and
   a fourth capacitor that is electrically coupled in series between the second signal line and the second input section.
(24) The biological signal detection device according to (23), in which the first amplifier circuit is configured to output a first signal based on a signal that is inputted via the third capacitor and a signal that is inputted via the fourth capacitor.
(25) The biological signal detection device according to (23) or (24), further including:
   a fifth resistor and a sixth resistor; and
   a second potential line that is electrically coupled to the third capacitor and the first input section via the fifth resistor, and is electrically coupled to the fourth capacitor and the second input section via the sixth resistor.
(26) The biological signal detection device according to any one of (1) to (22), further including:
   a first amplifier circuit including a first input section and a second input section;
   a second amplifier circuit including a third input section and a fourth input section;
   a third capacitor that is electrically coupled in series between the first signal line and the first input section;
   a fourth capacitor that is electrically coupled in series between the second signal line and the third input section; and
   a second potential line that is electrically coupled to the second input section and the fourth input section.
(27) A biological signal detecting system including:
   a measurement circuit including
      a first electrode and a second electrode that are configured to be in contact with a living body,
      a first signal line that is electrically coupled to the first electrode,
      a second signal line that is electrically coupled to the second electrode,
      a first resistor that is electrically coupled between the first signal line or the second signal line and a first potential line, and
      a first capacitor that is electrically coupled between the first signal line or the second signal line and the first potential line,
   the measurement circuit being configured to output a first signal based on a potential of the first signal line and a potential of the second signal line; and
   a signal processing circuit configured to generate a signal related to the living body, on the basis of the first signal outputted by the measurement circuit.

This application claims the benefit of Japanese Priority Patent Application JP2023-179964 filed with the Japan Patent Office on October 19, 2023, and Japanese Priority Patent Application JP2024-081761 filed with the Japan Patent Office on May 20, 2024, the entire contents of which are incorporated herein by reference.

It should be understood by those skilled in the art that various modifications, combinations, sub-combinations, and alterations may occur depending on design requirements and other factors insofar as they are within the scope of the appended claims or the equivalents thereof.

## Claims

1. A biological signal detection device comprising:
a first electrode and a second electrode that are configured to be in contact with a living body;
a first signal line that is electrically coupled to the first electrode;
a second signal line that is electrically coupled to the second electrode;
a first resistor that is electrically coupled between the first signal line or the second signal line and a first potential line; and
a first capacitor that is electrically coupled between the first signal line or the second signal line and the first potential line.

2. The biological signal detection device according to claim 1, further comprising a measurement circuit that includes the first electrode and the second electrode and is configured to generate a biological signal, wherein
the measurement circuit is configured to generate one signal as the biological signal with use of only the first electrode and the second electrode as electrodes configured to be in contact with the living body.

3. The biological signal detection device according to claim 2, wherein
the measurement circuit includes a first amplifier circuit that is electrically coupled to the first signal line and the second signal line, and
the first amplifier circuit is configured to output, as the biological signal, a first signal based on a potential of the first signal line and a potential of the second signal line.

4. The biological signal detection device according to claim 1, wherein a resistance value of the first resistor and a capacitance value of the first capacitor are set on a basis of a frequency of a signal that is inputted to the first signal line or the second signal line.

5. The biological signal detection device according to claim 1, wherein a cut-off frequency based on a resistance value of the first resistor and a capacitance value of the first capacitor is set on a basis of a frequency to be transmitted of a signal that is inputted to the first signal line or the second signal line.

6. The biological signal detection device according to claim 1, wherein a resistance value of the first resistor and a capacitance value of the first capacitor are determined on a basis of a frequency to be acquired of a brainwave.

7. The biological signal detection device according to claim 1, further comprising a first amplifier circuit configured to output a first signal based on a potential of the first signal line and a potential of the second signal line.

8. The biological signal detection device according to claim 7, wherein only the first electrode and the second electrode are provided, as electrodes configured to be in contact with the living body, for the first amplifier circuit.

9. The biological signal detection device according to claim 7, wherein the first amplifier circuit is configured to output the first signal based on differential amplification between the potential of the first signal line and the potential of the second signal line.

10. The biological signal detection device according to claim 7, wherein
the first amplifier circuit is electrically coupled to a power supply line, and
the first potential line comprises a wiring to which a potential between a potential of the power supply line and a ground potential is applied.

11. The biological signal detection device according to claim 7, wherein
the first amplifier circuit is electrically coupled to a power supply line, and
the first potential line comprises a wiring to which a ground potential is applied.

12. The biological signal detection device according to claim 1, wherein the first resistor and the first capacitor are each electrically coupled between the first signal line and the first potential line.

13. The biological signal detection device according to claim 1, wherein the first resistor and the first capacitor are electrically coupled in parallel to each other between the first signal line and the first potential line.

14. The biological signal detection device according to claim 13, further comprising a second resistor and a second capacitor that are electrically coupled in parallel to each other between the second signal line and the first potential line.

15. The biological signal detection device according to claim 1, wherein
the first resistor is electrically coupled in series between the first signal line and the first potential line, and
the first capacitor is electrically coupled in series between the second signal line and the first potential line.

16. The biological signal detection device according to claim 1, further comprising a first amplifier circuit including a first input section that is electrically coupled to the first electrode via the first signal line, a second input section that is electrically coupled to the second electrode via the second signal line, and an output section, wherein
the first amplifier circuit is configured to output a first signal based on a potential of the first signal line and a potential of the second signal line from the output section.

17. The biological signal detection device according to claim 16, wherein the first amplifier circuit is configured to output a second signal that is an inverted signal of the first signal.

18. The biological signal detection device according to claim 16, further comprising:
a third resistor that is electrically coupled between the second signal line and the second input section; and
a fourth resistor that is electrically coupled between the second input section and the output section.

19. The biological signal detection device according to claim 16, further comprising a second amplifier circuit configured to output a second signal based on the first signal and the potential of the second signal line.

20. The biological signal detection device according to claim 1, further comprising a current source configured to supply a current to the first signal line.

21. The biological signal detection device according to claim 20, wherein the current source is configured to supply an alternating current with a specific frequency to the first signal line.

22. The biological signal detection device according to claim 1, further comprising:
a third electrode configured to be in contact with the living body;
a third signal line that is electrically coupled to the third electrode;
a first amplifier circuit that is electrically coupled to the first signal line and the second signal line, and is configured to output a signal based on a potential of the first signal line and a potential of the second signal line; and
a third amplifier circuit that is electrically coupled to the second signal line and the third signal line, and is configured to output a signal based on the potential of the second signal line and a potential of the third signal line.

23. The biological signal detection device according to claim 1, further comprising
a first amplifier circuit including a first input section and a second input section;
a third capacitor that is electrically coupled in series between the first signal line and the first input section; and
a fourth capacitor that is electrically coupled in series between the second signal line and the second input section.

24. The biological signal detection device according to claim 23, wherein the first amplifier circuit is configured to output a first signal based on a signal that is inputted via the third capacitor and a signal that is inputted via the fourth capacitor.

25. The biological signal detection device according to claim 23, further comprising:
a fifth resistor and a sixth resistor; and
a second potential line that is electrically coupled to the third capacitor and the first input section via the fifth resistor, and is electrically coupled to the fourth capacitor and the second input section via the sixth resistor.

26. The biological signal detection device according to claim 1, further comprising:
a first amplifier circuit including a first input section and a second input section;
a second amplifier circuit including a third input section and a fourth input section;
a third capacitor that is electrically coupled in series between the first signal line and the first input section;
a fourth capacitor that is electrically coupled in series between the second signal line and the third input section; and
a second potential line that is electrically coupled to the second input section and the fourth input section.

27. A biological signal detecting system comprising:
a measurement circuit including
a first electrode and a second electrode that are configured to be in contact with a living body,
a first signal line that is electrically coupled to the first electrode,
a second signal line that is electrically coupled to the second electrode,
a first resistor that is electrically coupled between the first signal line or the second signal line and a first potential line, and
a first capacitor that is electrically coupled between the first signal line or the second signal line and the first potential line,
the measurement circuit being configured to output a first signal based on a potential of the first signal line and a potential of the second signal line; and
a signal processing circuit configured to generate a signal related to the living body, on a basis of the first signal outputted by the measurement circuit.
